# EUROPEAN PATENT APPLICATION

(11) **EP 1 491 539 A1**
(43) Date of publication of application: **29.12.2004**
(21) Application number: 03715613.0
(22) Date of filing: 28.03.2003
(51) Int. Cl.: C07D 401/12, C07D 471/04, C07D 495/04, A61K 31/4439, A61K 31/444, A61P 1/04, A61P 43/00

(54) **1-N-AMINOBENZIMIDAZOLE DERIVATIVES**

(30) Priority: 29.03.2002 JP 2002096143
(71) Applicant: Zeria Pharmaceutical Co., Ltd., Tokyo 103-8351 (JP)
(72) Inventor: NAGASAWA, Masaaki, ZERIA PHARMACEUTICAL CO., LTD., Osato-gun (JP); NISHIOKA, Hiroyasu, ZERIA PHARMACEUTICAL CO., LTD, Osato-gun (JP); ASAMI, Kazuyasu, ZERIA PHARMACEUTICAL CO., LTD., Osato-gun (JP); MIURA, Naoyoshi, ZERIA PHARMACEUTICAL CO., LTD., Osato-gun (JP); NAKAMURA, Hideki, ZERIA PHARMACEUTICAL CO., LTD., Osato-gun (JP); MORITA, Hitoshi, ZERIA PHARMACEUTICAL CO., LTD., Osato-gun (JP)
(74) Representative: Hartz, Nikolai F., Dr.
(86) International application number: PCT/JP2003/003996
(87) International publication number: WO 2003/082854

(57) **Abstract**

Provided are 1-N-aminobenzimidazole derivatives represented by the following formula (I): 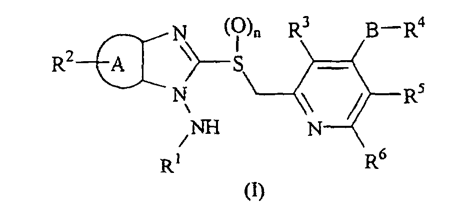 wherein R¹ and R² each represents a substituted or unsubstituted alkyl group or the like, R³, R⁵ and R⁶ each represents an alkyl group, alkoxy group, hydrogen atom or the like, R⁴ represents a substituted or unsubstituted alkyl group or the like, A represents a benzene ring or the like, B represents a hydrogen atom or the like, an n stands for an integer of from 0 to 2, or salts thereof; and medicines containing them.

The compounds (I) according to the present invention do not bring about much individual differences in therapeutic effects despite the existence of individual differences in the CYP2C19 activity. At the same dose, they can hence bring about appropriate therapeutic effects for all patients. In addition, they are low in the risk of induction of an interaction or a cancer caused by induction of the CYP1A family. Accordingly, they are useful as peptic ulcer therapeutic agents which are safe and surely bring about therapeutic effects.

## Description

### Technical Field

This invention relates to 1-N-aminobenzimidazole derivatives useful as peptic ulcer therapeutic agents, which have no individual differences in therapeutic effects and are high in safety, or as gastric acid secretion inhibitors in *Helicobacter pylori* eradication therapy.

### Background Art

Proton pump (H⁺/K⁺-ATPase) inhibitors are widely used as peptic ulcer therapeutic agents, as they strongly inhibit the secretion of gastric acid which is the major cause of peptic ulcer. Known as such proton pump inhibitors (hereinafter called the "conventional proton pump inhibitors") are omeprazole, esomeprazole, lansoprazole, rabeprazole, pantoprazole, and the like (JP-A-54-141783, WO94/27988, JP-A-61-50978, JP-A-64-6270, and JP-A-61-22079).

In recent years, it has been found from analyses of the kinetics of such drugs that the conventional proton pump inhibitors are metabolized primarily by CYP2C19, one of isoforms of cytochrome P450 (CYP) (Chin. Pharmacokinet., 31, 9-28 (1996); U.S. Patent No. 5,877,192; Aliment. Pharmacol. Ther., 15, 793-803 (2001)). It is also known that many of the conventional proton pump inhibitors induce the CYP1A2 family which is another isoformof cytochrome P450 (Xenobiotica, **27**(1), 1-9, (1997)).

In man, CYP2C19 is known to have genetic polymorphism. It is known that due to the existence of a poor metabolizer genetically deficient in activity and an extensive metabolizer having activity, the effectiveness exhibited in persons with the poor metabolizer may not be sufficiently shown in persons with the extensive metabolizer when the conventional proton pump inhibitors susceptible to metabolism by CYP2C19 are taken at usual dose (Ann. Intern. Med., 129, 1027-1030 (1998); Gastroenterology, 120(Suppl. 1), A-432 (2001) (#2203); and Gastroenterology, 120(Suppl. 1), A-435 (2001) (#2219)). It is, therefore, considered that to allow these medicines to sufficiently exhibit their effectiveness shown on persons with the poor metabolizer, they need to be taken at higher dose to persons with the extensive metabolizer. However, their administration at such high dose is not needed for persons with the poor metabolizer and increases the onset rate of side effects.

For the reasons mentioned above, it is considered to be effective that, upon taking a conventional proton pump inhibitor, the CYP2C19 genotype of the person who is going to take it is determined and a dose at which the medicine effectively acts is set exclusively for the person on the basis of the genotype (Aliment. Pharmacol. Ther., 13, 453-458 (1999)).

Further, the above-described conventional proton pump inhibitors include those tending to induce enzymes of the CYP1A family. When these enzymes are induced, the pharmacological activities of medicines such as theophylline and caffeine, which are metabolized by them, are lost at an early stage, thereby potentially resulting in a drug interaction that the intended therapeutic effects would be obtained (Eur. J. Clin. Pharmacol., **48,** 391-395 (1995)).

It is also known that, after some precarcinogens are taken in the body, they are metabolized and activated by the CYP1A subfamily to have oncogenicity. It is, therefore, considered that, when the CYP1A family is induced by the administration of a CYP1A-family-inducing proton pump inhibitor, the activation of these precarcinogens is heightened to potentially increase the onset of cancers (Gastroenterology, **99**, 737-747 (1990)).

With the foregoing in view, there is an outstanding desire for a proton pump inhibitor, small in individual differences in therapeutic effects, which take place due to individual differences in the CYP2C19 activity. At the same dose, they can hence bring about appropriate therapeutic effects for all patients. In addition, they are low in the risk of induction of an interaction or a cancer caused by induction of the CYP1A family. Accordingly, they are useful as peptic ulcer therapeutic agents which are safe and surely bring about therapeutic effects.

### Disclosure of the Invention

Therefore, the present inventors synthesized numerous compounds, and newly contrived and practiced a comprehensive screening that relies upon their proton pump inhibitory ef fect, CYP2C19 activity inhibitory ability and CYP1A2 inducing ability as indices. As a result, it has been found that 1-N-aminoimidazole derivatives, which have a new structure with an amino group substituted on the 1-position of the imidazole ring, and their salts have an excellent proton pump inhibitory effect, are not metabolized much by CYP2C19 and are low in the CYP1A2 inducing ability, and therefore, are useful as peptic ulcer therapeutic agents which have small individual differences in therapeutic effects and are high in safety, leading to the completion of the present invention.

The present invention, therefore, provides a 1-N-aminobenzoimidazole derivative represented by the following formula (I): 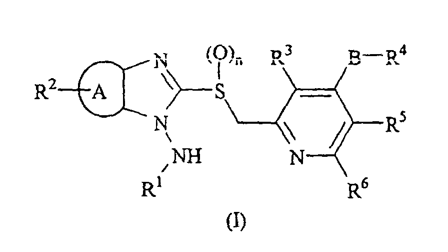 wherein:
R¹ represents an alkyl group optionally substituted by one or more substituents which may be the same or different and which are selected from halogen atoms, hydroxy groups, phenyl groups, hydroxyphenyl groups, amino groups, alkoxy groups, alkoxycarbonyl groups or alkylamino groups, an alkenyl group, an acyl group, an alkoxycarbonyl group, a benzyloxycarbonyl group, a formyl group, a phenyl group, or a hydrogen atom;
R² represents an alkyl group which may be substituted by a hydroxy or alkoxycarbonyl group, an acyl group which may be substituted by a halogen atom, a cyano group, a carboxyl group, an alkoxycarbonyl group, or a hydrogen atom;
R³, R⁵ and R⁶ may be the same or different and each represents an alkyl group, an alkoxy group or a hydrogen atom;
R⁴ represents an alkyl group optionally substituted by one or more substituents which may be the same or different and which are selected from halogen atoms, hydroxy groups, alkyl groups which may be substituted by 1 to 8 halogen atoms, alkoxy groups which may be substituted by 1 to 8 halogen atoms, furyl groups or morpholino groups, or a geranyl group;
A represents a benzene ring, a pyridine ring, or a thiophene ring;
B represents an oxygen atom or a sulfur atom; and
n stands for an integer of from 0 to 2; or a salt thereof.

The present invention also provides a medicine comprising a 1-N-aminobenzimidazole derivative represented by the formula (I) or a salt thereof.

The present invention also provides a medicinal composition comprising a 1-N-aminobenzimidazole derivative represented by the formula (I) or a salt thereof and a pharmacologically acceptable carrier.

The present invention also provides use of a 1-N-aminobenzimidazole derivative represented by the formula (I) or a salt thereof for the production of a medicine.

The present invention also provides a method for the treatment of a peptic ulcer, which comprises administering an effective amount of a 1-N-aminobenzimidazole derivative represented by the formula (I) or a salt thereof.

### Best Modes for Carrying out the Invention

In the present invention, each alkyl group, alkoxy group or the like is preferably in a linear, branched or cyclic form having 1 to 6 carbon atoms. On the other hand, each alkenyl group, acyl group or the like is preferably in a linear, branched or cyclic form having 2 to 6 carbon atoms.

The term "alkyl group" can include linear, branched or cyclic alkyl groups having 1 to 6 carbon atoms, for example, methyl, ethyl, propyl, isopropyl, cyclopropyl, butyl, isobutyl, sec-butyl, tert-butyl, cyclobutyl, pentyl, 1-methylbutyl, 2-methylbutyl, isopentyl, tert-pentyl, 1,2-dimethylpropyl, neopentyl, 1-ethylpropyl, cyclopentyl, hexyl, 1-methylpentyl, 2-methylpentyl, 3-methylpentyl, isohexyl, 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 2-methyl-1-ethylpropyl, 1-ethyl-2-methylpropyl, 1,1,2-trimethylpropyl, 1,2,2-trimethylpropyl, cyclohexyl, and the like. Among these, more preferred alkyl groups are linear or branched alkyl groups having 1 to 4 carbon atoms.

The term "alkoxy group" can include linear, branched or cyclic alkoxy groups having 1 to 6 carbon atoms, for example, methoxy, ethoxy, propoxy, isopropoxy, cyclopropoxy, butoxy, isobutoxy, sec-butoxy, tert-butoxy, cyclobutoxy, pentyloxy, 1-methylbutoxy, 2-methylbutoxy, isopentyloxy, tert-pentyloxy, 1,2-dimethylpropoxy, neopentyloxy, 1-ethylpropoxy, cylopentyloxy, hexyloxy, 1-methylpentyloxy, 2-methylpentyloxy, 3-methylpentyloxy, isohexyloxy, 1-ethylbutoxy, 2-ethylbutoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-methyl-1-ethylpropoxy, 1-ethyl-2-methylpropoxy, 1,1,2-trimethylpropoxy, 1,2,2-trimethylpropoxy, cyclohexyloxy, and the like. Among these, more preferred alkoxy groups are linear or branched alkoxy groups having 1 to 4 carbon atoms.

The term "alkenyl group" can include linear, branched or cyclic alkenyl groups having 1 to 3 double bonds and 2 to 6 carbon atoms, for example, vinyl, 1-propenyl, allyl, isopropenyl, 1-butenyl, 2-butenyl, 3-butenyl, isobutenyl, 1-pentenyl, 2-pentenyl, 3-pentenyl, 4-pentenyl, cyclopentenyl, 1-hexenyl, 2-hexenyl, 3-hexenyl, 4-hexenyl, 5-hexenyl, cyclohexenyl, 1,3-butadienyl, and the like. Among these, more preferred alkenyl groups are alkenyl groups having one double bond and 2 to 4 carbon atoms.

The term "acyl group" can include acyl groups having 2 to 6 carbon atoms, for example, acetyl, propionyl, butyryl, isobutyryl, valeryl, isovaleryl, pivaloyl, and the like. Among these, more preferred acyl groups are alkylcarbonyl groups having 2 to 5 carbon atoms in total.

The term "alkoxycarbonyl group" can include those formed by bonding a carbonyl group to the above-described C₁₋₆ alkoxy groups, for example, methoxycarbonyl, ethoxycarbonyl, propoxycarbonyl, isopropoxycarbonyl, cyclopropoxycarbonyl, butoxycarbonyl, isobutoxycarbonyl, sec-butoxycarbonyl, tert-butoxycarbonyl, cyclobutoxycarbonyl, pentyloxycarbonyl, 1-methylbutoxycarbonyl, 2-methylbutoxycarbonyl, isopentyloxycarbonyl, tert-pentyloxycarbonyl, 1,2-dimethylpropoxycarbonyl, neopentyloxycarbonyl, 1-ethylpropoxycarbonyl, cyclopentyloxycarbonyl, hexyloxycarbonyl, 1-methylpentyloxycarbonyl, 2-methylpentyloxycarbonyl, 3-methylpentyloxycarbonyl, isohexyloxycarbonyl, 1-ethylbutoxycarbonyl, 2-ethylbutoxycarbonyl, 1,1-dimethylbutoxycarbonyl, 1,2-dimethylbutoxycarbonyl, 1,3-dimethylbutoxycarbonyl, 2,2-dimethylbutoxycarbonyl, 2,3-dimethylbutoxycarbonyl, 3,3-dimethylbutoxycarbonyl, 1-methyl-1-ethylpropoxycarbonyl, 1-ethyl-2-methylpropoxycarbonyl, 1,1,2-trimethylpropoxycarbonyl, 1,2,2-trimethylpropoxycarbonyl, cyclohexyloxycarbonyl, and the like. Among these, more preferred alkoxycarbonyl groups are alkoxycarbonyl groups having 2 to 5 carbon atoms in total.

The term "alkylamino group" can preferably include those formed by substituting one or two of the hydrogen atoms of an amino group with the above-described C₁₋₆ alkyl groups, for example, methylamino, ethylamino, propylamino, isopropylamino, cyclopropylamino, butylamino, isobutylamino, pentylamino, isopentylamino, cyclopentylamino, hexylamino, cyclohexylamino, dimethylamino, diethylamino, dipropylamino, diisopropylamino, dicyclopropylamino, dibutylamino, diisobutylamino, dipentylamino, diisopentylamino, dicyclopentylamino, dihexylaminio, dicyclohexylamino, methylethylamino, methylpropylamino, methylisopropylamino, methylbutylamino, methylisobutylamino, methylpentylamino, methylhexylamino, ethylpropylamino, ethylisopropylamino, ethylbutylamino, ethylisobutylamino, ethylpentylamino, ethylhexylamino, propylisopropylamino, propylbutylamino, propylisobutylamino, propylpentylamino, propylhexylamino, isopropylbutylamino, isopropylisobutylamino, isopropylpentylamino, isopropylhexylamino, butylisobutylamino, butylpentylamino, butylhexylamino, isobutylpentylamino, isobutylhexylamino, pentylhexylamino, and the like. Among these, more preferred alkylamino groups are monoalkylamino groups having 1 to 4 carbon atoms.

In the present invention, the term "halogen atom" means a fluorine atom, a chlorine atom, a bromine atom or an iodine atom. Among these, a more preferred halogen atom is a fluorine atom.

Further, the term "hydroxyphenyl group" means one formed by substituting one hydroxyl group on a phenyl group. Examples include *o*-hydroxyphenyl (2-hydroxyphenyl or 6-hydroxyphenyl), *m*-hydroxyphenyl (3-hydroxyphenyl or 5-hydroxyphenyl), and *p*-hydroxyphenyl (4-hydroxyphenyl).

No particular limitation is imposed on the salt of the compound (I) according to the present invention, insofar as it is a pharmacologically acceptable salt. Examples include its acid addition salts with inorganic acids, such as its hydrochloride, sulfate, nitrate, phosphate, hydrobromide, hydroiodide and tetrafluoroborate; its addition salts with organic acids, such as its acetate, oxalate, malonate, succinate, maleate, fumarate, lactate, malate, citrate, tartrate, methanesulfonate and ethanesulfonate; and its metal salts such as its sodium salt, potassium salt, calcium salt and magnesium salt.

Preferred examples of the 1-N-aminobenzimidazole derivative (I) according to the present invention include compounds in which R¹ is an unsubstituted C₁₋₆ alkyl group, a C₁₋₆ alkyl group substituted by three halogen atoms, a C₁₋₆ alkyl group substituted by one hydroxy, phenyl or hydroxyphenyl group, a C₁₋₆ alkyl group substituted by one C₁₋₆ alkoxycarbonyl group, a C₁₋₆ alkyl group having one di (C₁₋₆ alkyl) amino group, a C₂₋₆ alkenyl group, an allyl group, or a phenyl group; R² is a C₁₋₆ alkyl group substituted by one hydroxyl group or C₁₋₆ alkoxycarbonyl group, a C₂₋₆ acyl group substituted by two halogen atoms, a cyano group, a carboxyl group, a C₁₋₆ alkoxycarbonyl group or a hydrogen atom; R³, R⁵ and R⁶ may be the same or different and are each a C₁₋₆ alkyl group, a C₁₋₆ alkoxy group or a hydrogen atom; R⁴ is a C₁₋₆ alkyl group optionally having one or more substituents which may be the same or different and are selected from halogen atoms, hydroxyl groups, C₁₋₆ alkyl groups which may be substituted by 1 to 8 halogen atoms, C₁₋₆ alkoxy groups which may be substituted by 1 to 8 halogen atoms, furyl groups or morpholino groups, or a geranyl group; A is a benzene ring; B is an oxygen atom; and n is 1. Particularly preferred are compounds in which R¹ is a methyl, ethyl, propyl, isopropyl, isobutyl, hexyl, 2-hydroxyethyl, 2-hydroxypropyl, 3-hydroxypropyl, 4-hydroxybutyl, 5-hydroxypentyl, 2,2,2-trifluoroethyl, 2-phenethyl, benzyl, allyl, p-hydroxybenzyl, 2-hydroxy-2-phenethyl, 2-dimethylaminoethyl, methoxycarbonylmethyl or phenyl group; R² is a methoxy, difluoromethoxy, cyano, methoxycarbonyl, methoxycarbonylmethyl, carboxyl or hydroxymethyl group, or a hydrogen atom; R³ is a methyl or methoxy group or a hydrogen atom; R⁴ is a methyl, ethyl, propyl, isopropyl, butyl, isobutyl, hexyl, octyl, 2-methoxyethyl, 3-methoxypropyl, 2,2,2-trifluoroethyl, 4,4,4-trifluorobutyl, 2,2,3,3,4,4,4-heptafluorobutyl, 2-(2,2,2-trifluoroethoxy)ethyl, 3-(2,2,2-trifluoroethoxy)propyl, 2-hydroxyethyl or geranyl group; R⁵ is a methyl group or a hydrogen atom; R⁶ is a hydrogen atom; A is a benzene ring; B is an oxygen atom; and n is 1.

There can be stereoisomers on the compounds according to the present invention. Their optically active isomers, racemic mixtures and diastereomers are all encompassed in the present invention. Further, solvatesrepresentedbyhydrates are also included in the compounds according to the present invention.

The compounds according to the present invention can be produced, for example, by a process to be shown next. 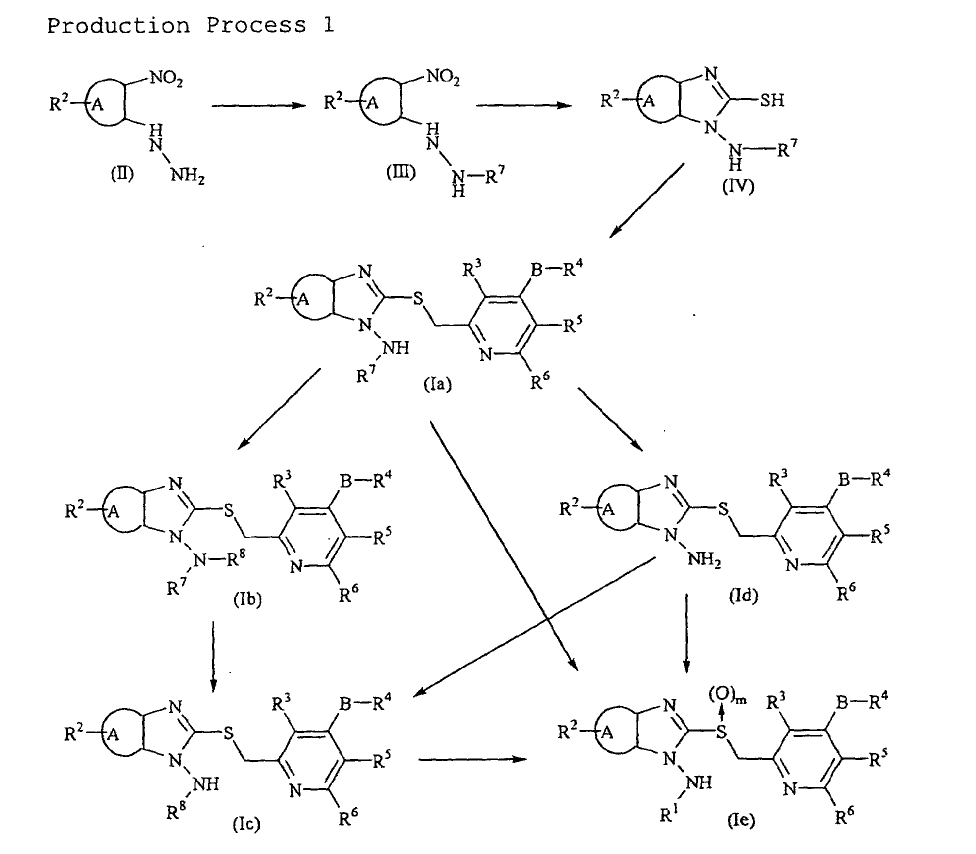 wherein R⁷ represents a benzyloxycarbonyl group or a tert-butoxycarbonyl group; R⁸ represents an alkyl group optionally having one or more substituent groups which may be the same or different and are selected from halogen atoms, hydroxy groups, phenyl groups, hydroxyphenyl groups, amino groups, alkoxy groups, alkoxycarbonyl groups or alkylamino groups, an alkenyl group, an acyl group, an alkoxycarbonyl group, a benzyloxycarbonyl group, a formyl group or a phenyl group; m stands for an integer of 1 or 2, and R¹, R², R³, R⁴, R⁵, R⁶, A and B have the same meanings as defined above.

Described specifically, a benzyloxycarbonyl group or tert-butoxycarbonyl group is added to the terminal amino group of a hydrazine derivative (II) to convert it into (III). The nitro group is then reduced, and in the presence of carbon disulfide and a base or in the presence of a complex of carbon disulfide, a base and a solvent, a mercaptoimidazole derivative (IV) is obtained. As the base for use in the reaction, sodium hydroxide, potassium hydroxide or the like can be employed. As the solvent, an alcohol such as methanol or ethanol can be employed. As the complex of carbon disulfide, the base and the solvent, a complex formed from carbon disulfide, ethanol and potassium hydroxide (for example, potassium ethylxanthate or the like) can be mentioned. The reaction is effected preferably at room temperature to reflux temperature for 1 to 24 hours under stirring.

Subsequently, the mercaptoimidazole (IV) and a 2-chloromethylpyridine are reacted in the presence of a base to yield an invention compound (Ia) having a benzyloxycarbonylamino group or a tert-butoxycarbonylamino group at the 1-N position of the imidazole ring. As the base for use in the reaction, lithium hydroxide, sodium hydroxide, potassium hydroxide or the like can be employed. As a solvent, an alcohol such as methanol or ethanol or a water-containing alcohol thereof, a aprotonic solvent such as dimethyl sulfoxide or N, N-dimethylformamide, or an ether such as tetrahydrofuran or dioxane can be employed. The reaction is conducted preferably at room temperature to reflux temperature for 1 to 24 hours under stirring.

The invention compound (Ia) can be converted by a reductive alkylation reaction into another invention compound (Ic) via (Id). The reaction is conducted by removing the benzyloxycarbonyl or tert-butoxycarbonyl group of (Ia) with an acid such as hydrochloric acid or trifluoroacetic acid or a base or in accordance with a catalytic reduction reaction in a solvent such as an ether, an alcohol or a water-containing alcohol to convert (Ia) into (Id), reacting (Id) with an aldehyde such as formaldehyde or acetaldehyde or a ketone such as acetone or methyl ethyl ketone in the presence of an acid catalyst, and then reducing the reaction product with a reducing agent. The solvent for use in the reaction can be an alcohol such as methanol or ethanol, an ether such as diethyl ether, diisopropyl ether, tetrahydrofuran or dioxane, acetonitrile, or a water-containing solvent thereof. As the acid catalyst, hydrochloric acid or the like can be used. As the reducing agent, sodium borohydride, sodium cyanoborohydride or the like can be used. The reaction can be conducted in a range of from 0°C to 60°C.

The invention compound (Ia) can also be converted into the invention compound (Ic) by conducting a substitution reaction to the amino group at the 1-N position middle of ring as needed, and then removing the benzyloxycarbonyl group or tert-butoxycarbonyl group. As the substitution reaction, a known alkylation reaction can be mentioned. The substitution reaction can be effected by reacting a corresponding alkyl halide or the like in the presence of a base such as sodium hydride or potassium hydride in a solvent such as dimethyl sulfoxide or N, N-dimethylformamide. The reaction is usually conducted in a range of from room temperature to 60°C.

Further, the invention compound (Ia) can also be converted into the invention compound (Ic) by conducting beforehand a substitution reaction to the amino group at the 1-N position to convert (Ia) into (Ib) and then removing the benzyloxycarbonyl group or tert-butoxycarbonyl group.

The invention compound (Ia, Ic or Id) obtained as described above can be converted into a further invention compound (Ie) by conducting an oxidation reaction. For the oxidation reaction, an organic peracid such as m-chloroperbenzoic acid or peracetic acid, sodium metaperiodate, an alcohol peroxide such as hydrogen peroxide solution, cumene hydroperoxide, and t-butoxy peroxide, OXONE (product of E. I. du Pont de Nemours & Co., Inc.), or the like can be used. The oxidation reaction is conducted preferably at a temperature of from 0 to 50°C for 10 minutes to 24 hours under stirring in a solvent such as methylene chloride, chloroform, N,N-dimethylformamide, toluene or ethylacetate or a mixed solvent thereof.

When it is desired to obtain each of the invention compounds in the form of an optically active isomer, the invention compound in the form of the desired optically active isomer can be obtained by using diisopropylethylamine, titanium tetraisopropoxide and an optically active tartrate (choose either the L- (+) isomer or D- (-) isomer as needed to obtain the desired optical isomer) and conducting oxidation with an alcohol peroxide (Sharpless oxidation). 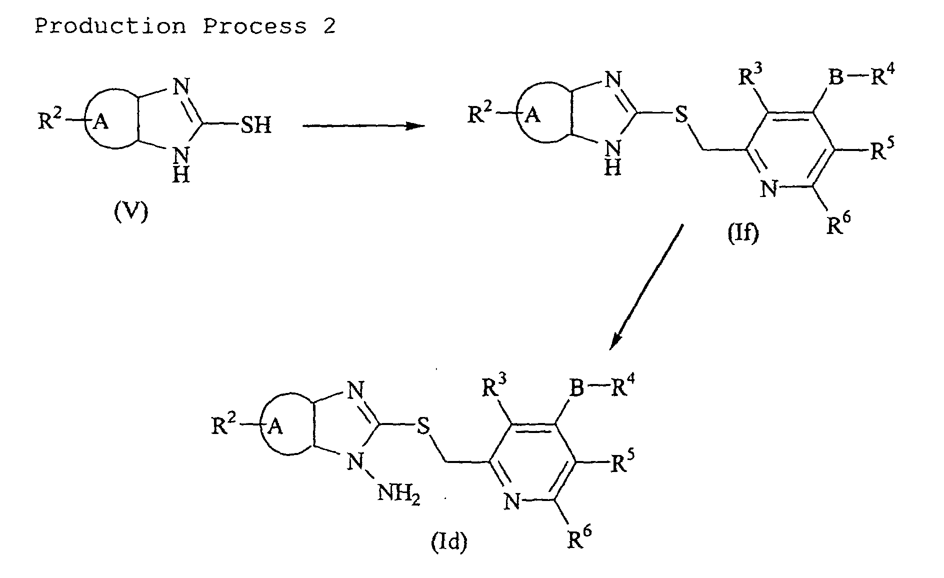 wherein R², R³, R⁴, R⁵, R⁶, A and B have the same meanings as defined above.

Described specifically, a mercaptoimidazole derivative (V) is coupled with a 2-chloromethylpyridine derivative in the presence of a base to afford an invention compound (If). The reaction is effected in a similar manner as in the above-described reaction between the mercaptoimidazole (IV) and the 2-chloromethylpyridine. Further, (If) can be converted into the invention compound (Id), which has an amino group at the 1-Nposition of an imidazole ring, by reacting (If) with N-tert-butoxycarbonyl-3-(4-cyanophenyl)oxaziridine and then conducting solvolysis in a manner known per se in the art. This reaction is conducted following a known process (J. Org. Chem., **58,** 4791 (1993) or Tetrahedron Lett., **36**, 1439 (1995)).

The invention compound (Id) obtained as described above can be converted to the further invention compound (Ie) by additionally conducting an oxidation reaction. The oxidation reaction is conducted in a similar manner as that described above. When it is desired to obtain each of the invention compound in the form of an optically active isomer, a procedure similar to that described above is performed.

The invention compound obtained as (Ia), (Ic), (Id) or (Ie) or as an optical isomer thereof can be converted into a desired salt in a manner known per se in the art by using an inorganic acid such as hydrochloric acid, sulfuric acid, nitric acid, phosphoric acid, hydrobromic acid, hydroiodic acid or tetrafluoroboric acid, an organic acid such as acetic acid, oxalic acid, malonic acid, succinic acid, maleic acid, fumaric acid, oxalic acid, malic acid, citric acid, tartaric acid, methanesulfonic acidor ethanesulfonic acid, or an alkali such as sodium hydroxide, potassium hydroxide, calcium hydroxide or magnesium hydroxide.

The compound (I) according to the present invention has characteristics that it is equipped with an excellent proton pump inhibitory effect and gastric acid secretion inhibitory effect, is not metabolized much by human CYP2C19 and is low in the CYP1A2 inducing ability. Accordingly, the compound (I) according to the present invention does not bring about much individual differences in therapeutic effects despite the existence of individual differences in the CYP2C19 activity. At the same dose, they can hence bring about appropriate therapeutic effects for all patients. In addition, they are low in the risk of induction of an interaction or a cancer caused by induction of the CYP1A family. Accordingly, they are useful as peptic ulcer therapeutic agents which are safe and surely bring about therapeutic effects.

The compound (I) according to the present invention is also useful as an acid secretion inhibitor in *Helicobacter* *pylori* eradication therapy.

Upon administering the compound (I) according to the present invention as a peptic ulcer therapeutic agent, it can be orally administered in the form of a powder, granules, capsules, a syrup or the like or it can be parenterally administered in the form of suppositories, an injection, an external preparation or an infusion preparation. Its dosage varies depending upon the severity of condition, the age, the ulcer type, etc., but in general, about 0.01 to 200 mg/kg, preferably 0.05 to 50 mg/kg, more preferably 0.1 to 10 mg/kg can be administered in one to several portions per day.

Upon formation into a pharmaceutical preparation, it can be produced in a manner known per se in the art by using a conventional carrier for pharmaceutical preparations.

Specifically, when an oral solidpreparation is desired, an excipient, and if necessary, a binder, disintegrator, lubricant, colorant, corrigent and the like are added to a basis, and the resulting mixture is formed into tablets, coated tablets, granules, a powder, capsules or the like in a manner known *per se* in the art.

Usable examples of the excipient include lactose, corn starch, glucose, sorbitol, crystalline cellulose, and silicon dioxide; usable examples of the binder include polyvinyl alcohol, polyvinyl ether, ethylcellulose, methylcellulose, gum arabic, tragacanth gum, gelatin, shellac, hydroxypropyl cellulose, hydroxypropyl starch, and polyvinylpyrrolidone; usable examples of the disintegrator include starch, agar, gelatin powder, crystalline cellulose, calcium carbonate, sodium hydrogencarbonate, calcium citrate, dextrin, or pectin; usable examples of the lubricant include magnesium stearate, talc, polyethylene glycol, silica, andhydrogenated vegetable oil; usable examples of the colorant include those permitted for addition to medicinal products; and usable examples of the corrigent include cacao powder, peppermint camphor, aromatic acids, pennyroyal oil, borneol, an cinnamon powder. Such oral solid preparations can be formed into enteric preparations by using a coating base such as hydropropylmethylcellulose phthalate, hydropropylmethylcellulose acetate succinate, cellulose acetate phthalate or a methacrylate copolymer. To these tablets and granules, sugar coating, gelatin coating or any other coating can be of course applied as needed without causing any problem.

When the preparation of an injection is desired, a pH regulator, buffer, stabilizer, solubilizer and the like are added as needed to a base, and the resulting mixture is formed into a subcutaneous, intramuscular or intravenous injection in a manner known *per se* in the art.

### Examples

The present invention will next be described in further detail on the basis of examples, although the present invention shall not be limited to them.

### Referential Example 1

### Synthesis of 1-amino-2-[[4-(2,2,2-trifluoroethoxy)-3-methylpyridin-2-yl]methylthio]benzimidazole

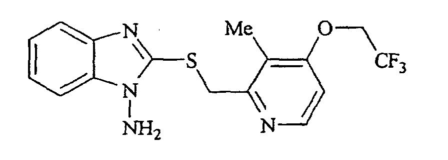

### Step 1

### Synthesis of 1,2-(2-tert-butyloxycarbonyl-hydrazino)nitrobenzene

2-Nitropheylhydrazine (15 g) was dissolved in 1,4-dioxane (150 mL). Under cooling with ice water, di(tert-butyl)dicarbonate (7.8 g), water (50 mL) andpotassium carbonate (17.6 g) were added, followed by stirring for 30 minutes. The reaction mixture was then stirred at room temperature for 12 hours. The reaction mixture was extracted with ethyl acetate. The organic layer was washed successively with a saturated aqueous solution of sodium bicarbonate, water, and a saturated aqueous solution of sodium chloride (hereafter called "brine"), and was then dried over with anhydrous sodium sulfate. The solvent was distilled off under reduced pressure, and the residue was purified by a silica gel chromatography (hexane:ethyl acetate = 10:1) to afford the title compound (19.9 g).
¹H-NMR(CDCl₃)δ: 1.47(9H,s),6.37(1H,bs),6.84-7.55(3H,m),8.1 8(1H,d),8.92(1H,s)

### Step 2

### Synthesis of 1-(N-tert-butyloxycarbonylamino)-2-meracaptobenzimidazole

1,2-(2-tert-Butyloxycarbonylhydrazino)nitrobenzene (25 g) was dissolved in ethanol (200 mL), followed by the addition of 10% palladium-charcoal (2.5 g). The resulting mixture was stirred under a hydrogen atmosphere at room temperature for 3 hours. After the mixture was filtered through celite, the filtrate was concentrated under reduced pressure. The concentrate was added to a mixture obtained by combining ethanol (150 mL), water (80 mL), carbon disulfide (40 mL) and potassium hydroxide (6.8 g) and stirring them at room temperature for 30 minutes, followed by heating under reflux. The pH of the reaction mixture is adjusted to 4 with acetic acid, and then the mixture is extracted with ethyl acetate. The organic layer was successively washed with water and brine and dried over anhydrous sodium sulfate, and subsequently, the solvent was distilled off. The residue was recrystallized from isopropyl ether to afford the title compound (23.5 g).
¹H-NMR(CDCl₃)δ: 1.53(9H,s),7.15-7.26(5H,m),9.72(1H,bs)

### Step 3

### Synthesis of 1-(N-tert-butyloxycarbonylamino)-2-[[4-(2,2,2-trifluoroethoxy)-3-methylpyridin-2-yl]methylthio]benzimidazole

1-(N-tert-Butyloxycarbonylamino)-2-mercaptobenzimidazole (75.6 g) was suspended in ethanol (700 mL), followed by the addition of 2-chloromethyl-3-methyl-4-(2,2,2-trifluoroethoxy)pyridine hydrochloride (78.7 g) and sodium hydroxide (24.0 g). The resulting mixture was stirred at 60°C for 1 hour. Water was added to the reaction mixture, and extraction was conducted with ethyl acetate. The organic layer was successively washed with a 1N aqueous solution of sodiumhydroxide, water andbrine, and was then dried over anhydrous sodium sulfate. The solvent was distilled off, and the residue was washed with isopropyl ether and then dried to afford the title compound (117.3 g).
¹H-NMR(CDCl₃)δ: 1.46(9H,bs),2.33(3H,s),4.38(2H,q),4.69(2H, s),6.63(1H,d),7.19-7.26(3H,m),7.56-7.59(1H,m),8.09(1H,bs), 8.32(1H,d)
Melting point: 153-155°C

### Step 4

### Synthesis of 1-amino-2-[[4-(2,2,2-trifluoroethoxy)-3-methylpyridin-2-yl]methylthio]benzimidazole

A 4N solution of hydrochloric acid in dioxane (700 mL) added to 1-(N-tert-butyloxycarbonylamino)-2-[[4-(2,2,2-trifluoroethoxy)-3-methylpyridin-2-yl]methylthio]benzimidazole (108.6 g), followed by stirring at 60°C for 30 minutes and then at room temperature for 1.5 hours. The reaction mixture was concentrated under reduced pressure, the residue was added into a saturated aqueous solution of sodium bicarbonate, and the solution was extracted with ethyl acetate. The organic layer was successively washed with water and brine, and was then dried over anhydrous sodium sulfate. The solvent was distilled off under reduced pressure, and the resulting crystals were washed with isopropyl ether and then dried to afford the title compound (68.0 g).
¹H-NMR (CDCl₃)δ: 2.34(3H,s),4.38(2H,q),4.74(2H, s),4.76(2H, s),6.63(1H,d),7.17-7.38(3H,m),7.63-7.67(1H,m),8.33(1H,d)
Melting point: 134-135°C

Compounds of Referential Examples 2 to 38 shown in Table 1 were then afforded by conducting a similar procedure as in Step 3 of Referential Example 1 except for the use of their corresponding chlorinated compounds instead of 2-chloromethyl-3-methyl-4-(2,2,2-trifluoroethoxy)pyridine hydrochloride and then repeating the procedure of Step 4.

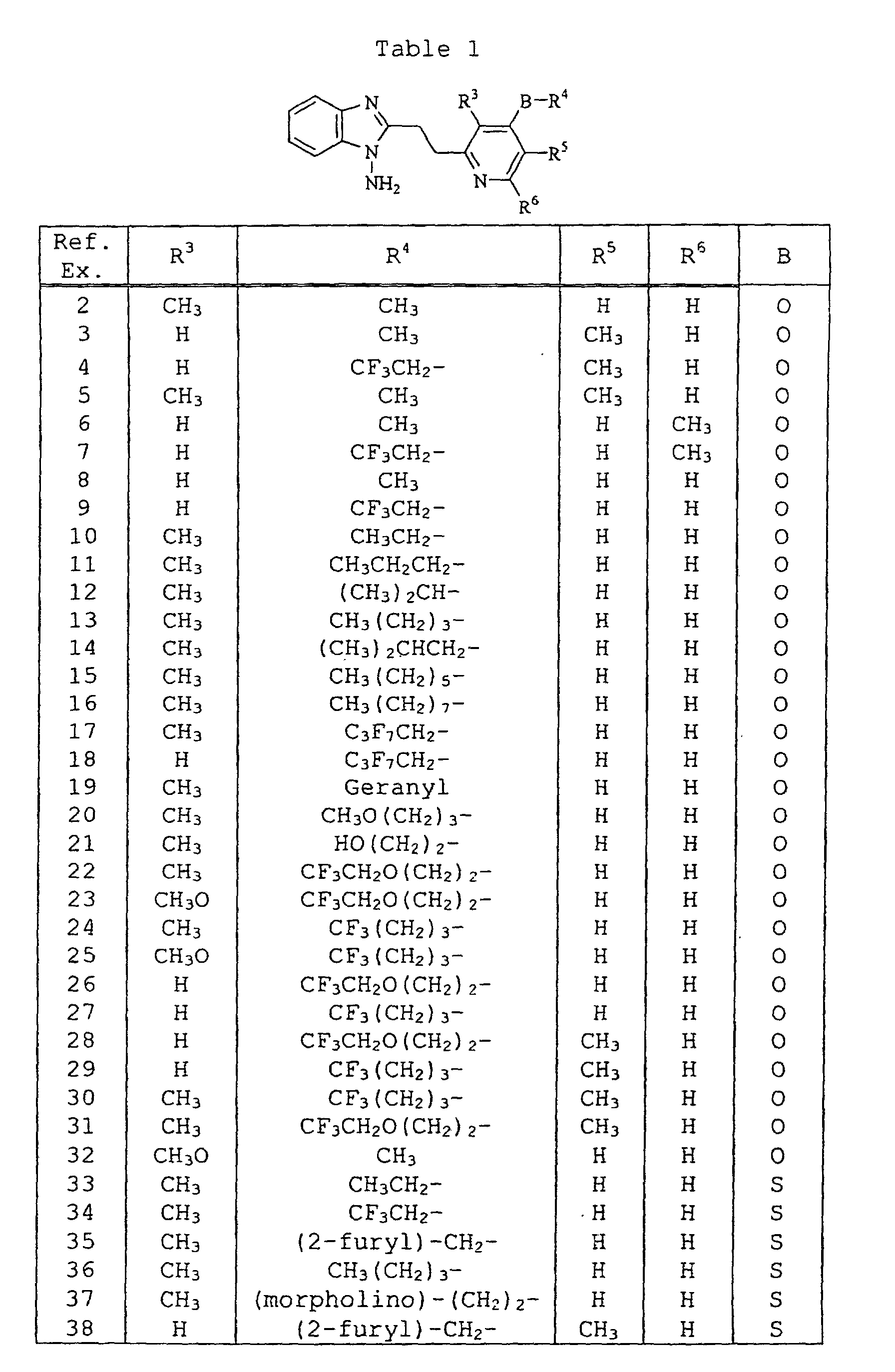

### Physicochemical Data

### Referential Example 2

¹H-NMR(CDCl₃)δ: 2.29(3H,s),3.86(3H,s),4.74(2H,s),4.75(2H,s),6.69(1H,d),7.17-7.24(2H,m),7.34-7.39(1H,m),7.62-7.68(1H,m),8.31(1H,d); IR(KBr,cm⁻¹): 3289,3135,1572,1435,1298,1271,1092; ; MS(FAB,MH⁺): 301; Melting point: 179-182°C

### Referential Example 3

¹H-NMR(CDCl₃)δ: 2.12(3H,s),3.77(3H,s),4.63(2H,s),4.66(2H,s),7.01(1H,s),7.18-7.37(3H,m),7.61-7.66(1H,m),8.17(1H,s); IR(KBr,cm⁻¹): 3119,1599,1435,1317,1155,1039; MS(FAB,MH⁺): 301; Melting point: 153-155°C

### Referential Example 4

¹H-NMR(CDCl₃)δ: 2.17(3H,s),4.26(2H,q),4.61(2H,s),4.62(2H,s),7.06(1H,s),7.19-7.36(3H,m),7.60-7.66(1H,m),8.25(1H,s); IR(KBr,cm⁻¹): 3346,1601,1269,1161,1072; MS(FAB,MH⁺): 369; Melting point: 183-184°C

### Referential Example 5

¹H-NMR(CDCl₃)δ: 2.24(3H,s),2.35(3H,s),3.74(3H,s),4.73(4H,s),7.20-7.39(3H,m),7.64-7.67(1H,m),8.20(1H,s); IR(KBr,cm⁻¹) : 3273,1591,1439,1265,1072; MS(FAB,MH⁺): 315; Melting point: 116-118°C

### Referential Example 6

¹H-NMR(CDCl₃)δ: 2.48(3H,s),3.74(3H,s),4.59(2H,s),4.71(2H,s),6.55(1H,d),6.88(1H,d),7.18-7.37(3H,m),7.63-7.66(1H,m); IR(KBr,cm⁻¹): 3191,1601,1441,1338,1153,1059; MS(FAB,MH⁺): 301; Melting point: 96-98°C

### Referential Example 7

¹H-NMR(CDCl₃)δ: 2.50(3H,s),4.26(2H,q),4.60(2H,s),4.66(2H,s),6.60(1H,d),6.96(1H,d),7.21-7.36(3H,m),7.61-7.67(1H,m);IR(KBr,cm⁻¹): 3323,1603,1435,1290,1170; MS(FAB,MH⁺): 369; Melting point: 120-122°C

### Referential Example 8

¹H-NMR (CDCl₃)δ: 3.78(3H,s),4.65(2H,s),4.67(2H,s),6.69(1H,dd),7.06(1H,d),7.18-7.36(3H,m),7.63-7.66(1H,m),8.36(1H,d);IR(KBr,cm⁻¹): 3107,1593,1317,1032; MS(FAB,MH⁺): 287; Melting point: 111-112°C

### Referential Example 9

¹H-NMR (CDCl₃)δ: 4.30(2H,q),4.64(2H,s),4.66(2H,s),6.76(1H,dd),7.17(1H,d),7.21-7.36(3H,m),7.62-7.66(1H,m),8.43(1H,d); IR(KBr,cm⁻¹): 3333,1599,1439,1292,1184,1084; MS(FAB,MH⁺): 355; Melting point: 118-120°C

### Referential Example 10

¹H-NMR (CDCl₃)δ: 1.45(3H,t),2.29(3H,s),4.07(2H,q),4.75(4H,s),6.66(1H,d),7.17-7.39(3H,m),7.63-7.69(1H,m),8.28(1H,d); IR(KBr,cm⁻¹): 1576,1433,1392,1302,1273,1080; MS(FAB,MH⁺):315; Melting point: 132-133°C

### Referential Example 11

¹H-NMR (CDCl₃)δ: 1.06(3H,t),1.81-1.88(2H,m),2.29(3H,s),3.96(2H,t),4.74(2H,s),4.75(2H,s),6.67(1H,d),7.18-7.39(3H,m),7.64-7.68(1H,m),8.29(1H,d); IR(KBr,cm⁻¹): 1585,1489,1385,1298,1269,1084; MS(FAB,MH⁺): 329; Melting point: 100-101°C

### Referential Example 12

¹H-NMR (CDCl₃)δ: 1.36(6H,d),2.26(3H,s),4.56-4.6,5(1H,m),4.74(2H,s),4.75(2H,s),6.66(1H,d),7.19-7.39(3H,m),7.63-7.68(1H,m),8.26(1H,d); IR(KBr,cm⁻¹): 1576,1442,1387,1298,1271,1115; MS(FAB,MH⁺): 329; Melting point: 117-119°C

### Referential Example 13

¹H-NMR (CDCl₃)δ: 0.99(3H,t),1.44-1.86(4H,m),2.29(3H,s),4.00(2H,t),4.74(4H,s),6.67(1H,d),7.18-7.39(3H,m),7.63-7.69(1H,m),8.28(1H,d); IR(KBr,cm⁻¹): 1576,1460,1435,1296,1278,1086; MS(FAB,MH⁺): 343; Melting point: 102-103°C

### Referential Example 14

¹H-NMR (CDCl₃)δ: 1.04(6H,d),2.06-2.18(1H,m),2.30(3H,s),3.76(2H,d),4.74(2H,s),4.75(2H,s),6.65(1H,d),7.18-7.38(3H,m),7.62-7.68(1H,m),8.28(1H,d); IR(KBr,cm⁻¹): 1580,1437,1385,1298,1271,1091; MS(FAB,MH⁺): 343; Melting point: 114-116°C

### Referential Example 15

¹H-NMR (CDCl₃)δ: 0.91(3H,t),1.31-1.47(6H,m),1.76-1.87(2H,m),2.29(3H,s),3.99(2H,t),4.74(2H,s),4.75(2H,s),6.67(1H,d),7.18-7.39(3H,m),7.63-7.68(1H,m),8.28(1H,d); IR(KBr,cm⁻¹):1578,1433,1381,1294,1273,1086; MS(FAB,MH⁺): 371; Melting point: 98-100°C

### Referential Example 16

¹H-NMR (CDCl₃)δ: 0.89(3H,t),1.26-1.47(10H,m),1.76-1.87(2H,m),2.29(3H,s),3.99(2H,t),4.74(2H,s),4.75(2H,s),6.67(1H,d),7.18-7.39(3H,m),7.64-7.68(1H,m),8.28(1H,d); IR(KBr,cm⁻¹):1578,1464,1433,1311,1275,1088; MS (FAB,MH⁺): 399; Melting point: : 97-98°C

### Referential Example 17

¹H-NMR (DMSO-d₆)δ: 2.25(3H,s),4.88(2H,s),5.01(2H,t),6.01(2H,s),7.10-7.21(3H,m),7.38(1H,d),7.51(1H,d),8.33(1H,d); IR(KBr,cm⁻¹): 3341,1578,1298; MS(FAB,MH⁺): 469; Melting point: 120-121°C

### Referential Example 18

¹H-NMR (CDCl₃)δ: 4.37-4.47(2H,m),4.66(4H,s),6.75(1H,dd),7.19-7.36(4H,m),7.59-7.65(1H,m),8.43(1H,d); IR(KBr,cm⁻¹): 3325,1574,1439,1306,1238,1120; MS(FAB,MH⁺): 455; Melting point: 92-93°C

### Referential Example 19

¹H-NMR (CDCl₃)δ: 1.61(3H,t),1.67(3H,s),1.73(3H,s),2.06-2.12(4H,m),2.29(3H,s),4.59(2H,d),4.74(2H,s),4.75(2H,s),5.06-5.08(1H,m),5.44(1H,dd),6.67(1H,d),7.18-7.39(3H,m),7.63-7.67(1H,m),8.28(1H,d); IR(KBr,cm⁻¹): 1579,1444,1396,1298,1273,1088; MS(FAB,MH⁺): 423; Melting point: 84-85°C

### Referential Example 20

¹H-NMR (CDCl₃)δ: 2.03-2.13(2H,m),2.29(3H,s),3.36(3H,s),3.56(2H,t),4.10(2H,t),4.75(4H,s),6.70(1H,d),7.20-7.39(3H,m),7.64-7.67(1H,m),8.29(1H,d); MS(FAB,MH⁺): 358

### Referential Example 21

¹H-NMR(DMSO-d₆)δ: 2.26(3H,s),3.70-3.80(2H,m),4.09(2H,t),4.65(2H,s),4.82-4.92(1H,m),5.98(2H,s),6.97(1H,d),7.12-7.19(2H,m),7.36-7.52(2H,m),8.25(1H,d)

### Referential Example 22

¹H-NMR (CDCl₃)δ: 2.31(3H,s),3.97(2H,q),4.01-4.05(2H,m),4.17-4.20(2H,m),4.74(2H,s),4.76(2H,s),6.68(1H,d),7.21-7.36(3H,m),7.64-7.67(1H,m),8.31(1H,d); MS(EI,M⁺): 428
Optical isomers: [α]_{D} of the (+) isomer = +82(c=0.33,CHCl₃) ; [α]_{D} of the (-) isomer = -77(c=0.32,CHCl₃)

### Referential Example 23

¹H-NMR (CDCl₃)δ: 3.94(2H,q),3.95(3H,s),4.02-4.05(2H,m),4.21-4.24(2H,m),4.76(2H,s),4.77(2H,s),6.76(1H,d),7.20-7.36(3H,m),7.64-7.70(1H,m),8.19(1H,d); MS(EI,M⁺): 444
Optical isomers: [α]_{D} of the (+) isomer = +55(c=0.27,CHCl₃) ; [α]_{D} of the (-) isomer = -56(c=0.26,CHCl₃)

### Referential Example 24

¹H-NMR (CDCl₃)δ: 2.09-2.38(4H,m),2.30(3H,s),4.06(2H,t),4.74(2H,s),4.76(2H,s),6.65(1H,d),7.21-7.39(3H,m),7.64-7.67(1H,m),8.30(1H,d)

### Referential Example 25

¹H-NMR (CDCl₃)δ: 2.09-2.39(4H,m),3.91(3H,s),4.11(2H,t),4.76(2H,s),4.77(2H,s),6.74(1H,d),7.18-7.39(3H,m),7.64-7.67(1H,m),8.19(1H,d); MS(EI,M⁺): 428
Optical isomers: [α]_{D} of the (+) isomer = +60(c=0.29,CHCl₃) ; [α]_{D} of the (-) isomer = -63(c=0.31,CHCl₃)

### Referential Example 26

¹H-NMR (CDCl₃)δ: 3.89(2H,q),3.92-3.96(2H,m),4.10-4.13(2H,m),4.65(2H,s),4.66(2H,s),6.71(1H,dd),7.07(1H,d),7.21-7.37(3H,m),7.63-7.66(1H,m),8.38(1H,d)

### Referential Example 27

¹H-NMR (CDCl₃)δ: 2.01-2.32(4H,m),3.98(2H,t),4.64(2H,s),4.65(2H,s),6.68(1H,dd),7.04(1H,d),7.22-7.36(3H,m),7.63-7.67(1H,m),8.37(1H,d)

### Referential Example 28

¹H-NMR (CDCl₃)δ: 2.14(3H,s),3.89(2H,q),3.92-3.96(2H,m),4.07-4.09(2H,m),4.61(2H,s),4.64(2H,s),7.00(1H,s),7.21-7.38(3H,m),7.60-7.64(1H,m),8.20(1H,s)

### Referential Example 29

¹H-NMR (CDCl₃)δ: 1.99-2.25(4H,m),2.12(3H,s),3.96(2H,t),4.61(2H,s),4.63(2H,s),6.97(1H,s),7.21-7.38(3H,m),7.62-7.66(1H,m),8.20(1H,s)

### Referential Example 30

¹H-NMR (CDCl₃)δ: 2.01-2.45(4H,m),2.22(3H,s),2.33(3H,s),3.83(2H,t),4.72(2H,s),4.73(2H,s),7.21-7.35(3H,m),7.62-7.64(1H,m),8.21(1H,s)

### Referential Example 31

¹H-NMR (CDCl₃)δ: 2.24(3H,s),2.36(3H,s),3.91-4.01(6H,m),4.72(2H,s),4.73(2H,s),7.20-7.35(3H,m),7.62-7.65(1H,m),8.21(1H,s)

### Referential Example 32

¹H-NMR (CDCl₃)δ: 1.41(3H,t),2.39(3H,s),2.98(2H,q),4.74(2H,bs),4.79(2H,s),6.99(1H,d),7.19-7.67(9H,m),8.25(1H,d); IR(KBr,cm⁻¹): 1566,1439,1275; MS(EI,M⁺): 330; Melting point: 92-96°C

### Referential Example 33

¹H-NMR (CDCl₃)δ: 2.49(3H,s),3.56(2H,q),4.71(2H,s),4.81(2H,s),7.11(1H,d),7.21-7.38(3H,m),7.62-7.68(1H,m),8.33(1H,d); IR(KBr,cm⁻¹): 3350,1620,1309,1271,1180,1080; MS(FAB,MH⁺):385; Melting point: 138-139°C

### Referential Example 34

¹H-NMR (CDCl₃)δ: 2.40(3H,s),4.19(2H,s),4.71(2H,s),4.79(2H,s),6.26-6.33(2H,m),7.09(1H,d),7.19-7.38(4H,m),7.64-7.67(1H,m),8.27(1H,d); IR(KBr,cm⁻¹): 3649,1560,1435,1269,1008; MS(EI,M⁺): 382; Melting point: 143-146°C

### Referential Example 35

¹H-NMR (CDCl₃)δ: 0.97(3H,t),1.48-1.75(4H,m),2.39(3H,s),2.94(2H,t),4.72(2H,s),4.79(2H,s),6.98(1H,d),7.19-7.38(3H,m),7.64-7.67(1H,m),8.26(1H,d); IR(KBr,cm⁻¹): 1560,1430,1340,1271; MS(EI,M⁺): 358; Melting point: 134-135°C

### Referential Example 36

¹H-NMR (CDCl₃)δ: 2.41(3H,s),2.50-2.54(4H,m),2.67-2.73(2H,m),3.07-3.12(2H,m),3.72-3.75(4H,m),4.73(2H,s),4.79(2H,s),7.01(1H,d),7.19-7.39(3H,m),7.62-7.68(1H,m),8.27(1H,d); IR(KBr,cm⁻¹): 3339,1560,1437,1273,1109; MS(EI,M⁺): 415; Melting point: 92-93°C

### Referential Example 37

¹H-NMR (CDCl₃)δ: 2.19(3H,s),4.09(2H,s),4.62(2H,s),4.63(2H,s),6.23-6.28(2H,m),7.27-7.24(2H,m),7.31-7.37(2H,m),7.49(1H,s),7.61-7.66(1H,m),8.18(1H,s); IR(KBr,cm⁻¹): 3330,1575,1432,1270,1090; MS(FAB,MH⁺): 383; Melting point: 129-132°C

### Referential Example 38

¹H-NMR (CDCl₃)δ: 3.90(6H,s),4.76(2H,s),4.80(2H,s),6.77(1H,d),7.17-7.38(3H,m),7.63-7.67(1H,m),8.19(1H,d); IR(KBr,cm⁻¹): 1583,1439,1304,1271,1064,1003; MS(FAB,MH⁺): 317; Melting point: 147-148°C

### Referential Example 39

### Synthesis of 1-(N-acetylamino)-2-[[4-(2,2,2-trifluoroethoxy)-3-methylpyridin-2-yl]methylthio]benzimidazole

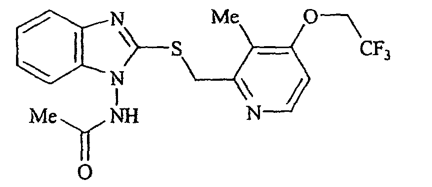

1-Amino-2-[[4-(2,2,2-trifluoroethoxy)-3-methylpyridin-2-yl]methylthio]benzimidazole (1.5 g) was dissolved in methylene chloride (30 mL), followed by the addition of acetic anhydride (1.6g) andpyridine (322mg). The resulting mixture was heated under reflux for 12 hours. A saturated aqueous solution of sodium bicarbonate was added to the reaction mixture to neutralize the same, and extraction was then effected with chloroform. The organic layer was successively washed with water and brine, and then dried over anhydrous sodium sulfate. The solvent was distilled off under reduced pressure, and the residue was purified by a silica gel chromatography (silica gel: "NH-DM1020" (product of FUJI SILYSIA CHEMICAL LTD.), chloroform:methanol = 30:1). Recrystallization was then conducted from a mixed solvent of hexane and ethyl acetate to afford the title compound (1.6 g).
¹H-NMR(DMSO-d₆)δ:
2.12(3H,s),2.26(3H,s),4.73(2H,s),4.90(2H,q),4.94(1H,d),7. 10(1H,d),7.18-7.25(3H,m),7.57-7.59(1H,m),8.31(1H,d),11.33 (1H,s)
IR (KBr, cm⁻¹): 3235,1674,1570,1446,1157
MS(EI,M⁺): 410
Melting point: 172-173°C

Compounds of Referential Examples 40 and 41 shown in Table 2 were then afforded by conducting a similar procedure as in Referential Example 39 except for the use of their corresponding acid anhydrides instead of acetic anhydride.

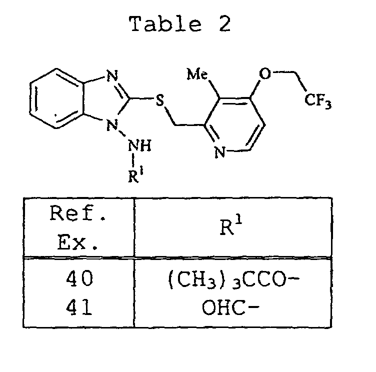

### Physicochemical Data

### Referential Example 40

¹H-NMR(CDCl₃)δ: 1.40(9H,s),2.32(3H,s),4.36(2H,q),4.69(2H,s),6.61(1H,d),7.08-7.23(3H,m),7.63-7.66(1H,m),8.28(1H,d),9.02(1H,s); IR(KBr,cm⁻¹): 1691,1581,1271,1165; MS(EI,M⁺): 452; Melting point: 200-201°C

### Referential Example 41

¹H-NMR(DMSO-d₆)δ: 2.25(3H,s),4.74(2H,s),4.90(2H,q),7.09(1H,d),7.20-7.28(3H,m),7.63-7.65(1H,m),8.31(1H,d),8.48(1H,s),11.51(1H,s); IR(KBr,cm⁻¹): 1709,1581,1452,1257,1161; MS(FAB,MH⁺): 397; Melting point: 200-201°C

### Referential Example 42

### Synthesis of 1-[N-(3-hydroxypropyl)amino]-2-[[4-(2,2,2-trifluoroethoxy)-3-methylpyridin-2-yl]methylthio]benzimidazole

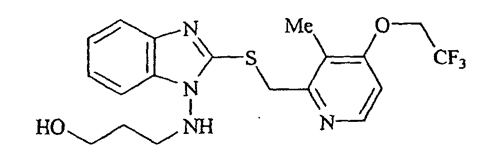

### Production Process A

1-Amino-2-[[4-(2,2,2-trifluoroethoxy)-3-methylpyridin-2-yl]methylthio]benzimidazole (1.5 g) was dissolved in methanol (15 mL), followed by the addition of malonyl aldehyde bisdimethylacetal ( 6 . 68 g) , conc. hydrochloric acid (0.5 mL) and water (3 mL). The resulting mixture was stirred at room temperature for 12 hours. The mixture was then ice-cooled, sodium borohydride (924 mg) was added, and stirring was conducted at the same temperature for 1 hour. Water was added to the reaction mixture, and extraction was conducted with ethyl acetate. The organic layer was washed with brine, and then dried over anhydrous sodium sulfate. The solvent was distilled off under reduced pressure, and the residue was purified by chromatography on a silica gel column (silica gel: "NH-DM1020" (product of FUJI SILYSIA CHEMICAL LTD.), chloroform:methanol = 50:1) to afford the title compound (540 mg).
¹H-NMR(CDCl₃)δ: 1.80-1.84(2H,m),2.36(3H,s),2.42-2.65(1H,bs),3.36(2H,q),3.82-3.86(2H,m),4.38(2H,q),4.77(2H,s),5.25(1H,t),6.65(1H,d),7.15-7.25(2H,m),7.30-7.40(1H,m),7.62-7.72(1H,m),8.35(1H,d)
IR(KBr,cm⁻¹): 3241,3065,2930,2901,1586,1476,1456,1435,1311,1287,1271,1163,1119,1082,976,963,747
MS(FAB,MH⁺): 427
Melting point: 113-114°C

### Production Process B

### Step B1

### Synthesis of 3-benzoyloxy-1-bromopropane

3-Bromopropanol (34.6 g) was dissolved in 1,2-dichloroethane (300 mL), followed by the addition of pyridine (19.7 g) and 4-dimethylaminopyridine (3.0 g). Benzoyl chloride (35.0 g) was then added dropwise under ice cooling, and the resultant mixture was stirred at 60°C for 2 hours. The reaction mixture was poured into ice water. The organic layer was successively washed with 1N hydrochloric acid, brine and water, and then dried over anhydrous sodium sulfate. The solvent was distilled off under reduced pressure, and the residue was purified by chromatography on a silica gel column (silica gel: "NH-DM1020" (product of FUJI SILYSIA CHEMICAL LTD.), hexane:ethyl acetate = 20:1) to afford the title compound (43.0 g).
¹H-NMR(CDCl₃)δ: 2.29-2.38(2H,m),3.51-3.60(2H,m),4.48(2H,t),7.42-7.60(3H,m),8.02-8.06(2H,m)

### Step B2

### Synthesis of 1-[N-tert-butyloxycarbonyl-N-(3-benzoyloxypropyl)amino]-2- [[4-(2,2,2-trifluoroethoxy)-3-methylpyridin-2-yl]methylthio]benzimidazole

1-(N-tert-Butyloxycarbonylamino)-2-[[4-(2,2,2-trifluoroethoxy)-3-methylpyridin-2-yl]methylthio]benzimidazole (1.0 g) was dissolved in N,N-dimethylformamide (20 mL), followed by the addition of 60% sodium hydride (80 mg) under ice cooling. The resulting mixture was stirred at room temperature for 30 minutes. 3-Benzoyloxy-1-bromopropane (520 mg), which had been obtained in Step 1, was then added, followed by stirring at 50°C for 1 hour. The reaction mixture was poured into ice water and extracted with ethyl acetate. The organic layer was successively washed with water and brine, and then dried over anhydrous sodium sulfate. The solvent was distilled off under reduced pressure, and the residue was purified by a silica gel chromatography(n-hexane:ethyl acetate = 1:1) to afford the title compound (940 mg).
¹H-NMR(CDCl₃)δ: 1.23-1.28(9H,bs),2.06-2.12(2H,m),2.30(3H,s),3.92-4.05(2H,m),4.35-4.39(2H,m),4.38(2H,q),4.83(2H,s),6.70(1H,d),7.20-7.71(7H,m),7.70-8.00(2H.m),8.29(1H,d)

### Step B3

### Synthesis of 1-[N-(3-hydroxypropyl)amino]-2-[[4-(2,2,2-trifluoroethoxy)-3-methylpyridin-2-yl]methylthio]benzimidazole

1-[N-tert-Butyloxycarbonyl-N-(3-benzoyloxypropyl)amino]-2-[[4-(2,2,2-trifluoroethoxy)-3-methylpyridin-2-yl]methylthio]benzimidazole (930 mg) was dissolved in methanol (10mL) , followed by the addition of conc. hydrochloric acid (3 mL) and water (3 mL) under cooling with ice water. The resulting mixture was stirred at 60°C for 1 hour. The mixture was neutralized with a saturated aqueous solution of sodium bicarbonate, and extracted with ethyl acetate. The organic layer was concentrated under reduced pressure, and the residue was dissolved in methanol (5 mL). To the solution, sodium hydroxide (160 mg) and water (5 mL) was added, followed by stirring at 60°C for 1 hour. Water was added to the reaction mixture, and the mixture was xtracted with ethyl acetate. The organic layer was successively washed with water and brine, and then dried over anhydrous sodium sulfate. The solvent was distilled off under reduced pressure, and the residue was recrystallized from isopropyl ether. The resultant crystals were collected by filtration, and then dried to afford the title compound (450 mg).
¹H-NMR(CDCl₃)δ: 1.80-1.84(2H,m),2.36(3H,s),2.42-2.65(1H,bs),3.36(2H,q),3.82-3.86(2H,m),4.38(2H,q),4.77(2H,s),5.25(1H,t),6.65(1H,d),7.15-7.25(2H,m),7.30-7.40(1H,m),7.62-7.72(1H,m),8.35(1H,d)
Melting point: 113-114°C

### Referential Example 43

### Synthesis of 1-[N-(2-hydroxyethyl)amino]-2-[[4-(2,2,2-trifluoroethoxy)-3-methylpyridin-2-yl]methylthio]benzimidazole

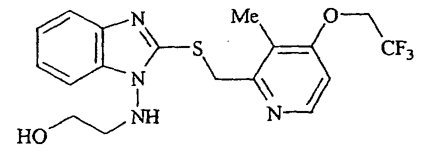

1-Amino-2-[[4-(2,2,2-trifluoroethoxy)-3-methylpyridin-2-yl]methylthio]benzimidazole (1.0 g) was dissolved in methanol (10 mL), followed by the addition of glycoaldehyde dimer (650 mg) and conc. hydrochloric acid (0.6 mL). The resulting mixture was stirred at room temperature for 3 hours. The mixture was then ice-cooled, sodium borohydride (616 mg) was added, and the thus-obtained mixture was stirred at the same temperature for 1 hour. Water was added to the reaction mixture, and extraction was conducted with ethyl acetate. The organic layer was washed with brine, and then dried over anhydrous sodium sulfate. The solvent was distilled off under reduced pressure, and the residue was purified by chromatography on a silica gel column (silica gel: "NH-DM1020" (product of FUJI SILYSIA CHEMICAL LTD.), chloroform:methanol = 20:1) to afford the title compound (640 mg).
¹H-MMR(CDCl₃)δ: 2.36(3H,s),2.74(1H,t),3.3-3.42(2H,m),3.64-3.70(2H,m),4.40(2H,q),4.79(2H,s),5.18(1H,t),6.65(1H,d),7.21-7.69(4H,m),8.35(1H,d)

### Referential Example 44

### Synthesis of 1-[N-(2-methylpropyl)amino]-2-[[4-(2,2,2-trifluoroethoxy)-3-methylpyridin-2-yl]methylthio]benzimidazole

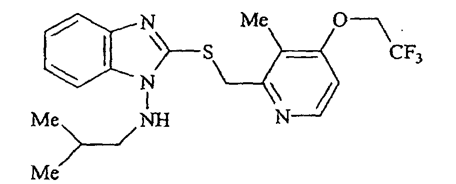

1-Amino-2-[[4-(2,2,2-trifluoroethoxy)-3-methylpyridin-2-yl]methylthio]benzimidazole (5.0 g) was dissolved in methanol (30 mL), followed by the addition of isobutyl aldehyde (1.95 g) and conc. hydrochloric acid (0.1 mL). The resulting mixture was stirred at 50°C for 1 hour. The mixture was then ice-cooled, sodium borohydride (1.54 g) was added, and the thus-obtained mixture was stirred at the same temperature for 1 hour. Waterwas added to the reactionmixture, and extraction was conducted with ethyl acetate. The organic layer was successively washed with a saturated aqueous solution of sodium bicarbonate and brine, and then dried over anhydrous sodium sulfate. The solvent was distilled off under reduced pressure, and the residue was purified by chromatography on a silica gel column (hexane:ethyl acetate = 2:1) to afford the title compound (4.7 g).
¹H-NMR(CDCl₃)δ:
1.03(6H,d),1.78-1.88(1H,m),2.35(3H,s),3.01(2H,t),4.40(2H,q),4.75-4.78(1H,m),4.79(2H,s),6.65(1H,d),7.19-7.36(3H,m),7.65-7.69(1H,m),8.37(1H,d)
IR(KBr,cm⁻¹): 3213,1579,1425,1304,1267,1165,1109
MS(EI,M⁺): 424
Melting point: 82-83°C

The procedures of Referential Examples 42-44 were then selectively performed to afford compounds of Referential Examples 45-70 shown in Table 3 and Table 4.

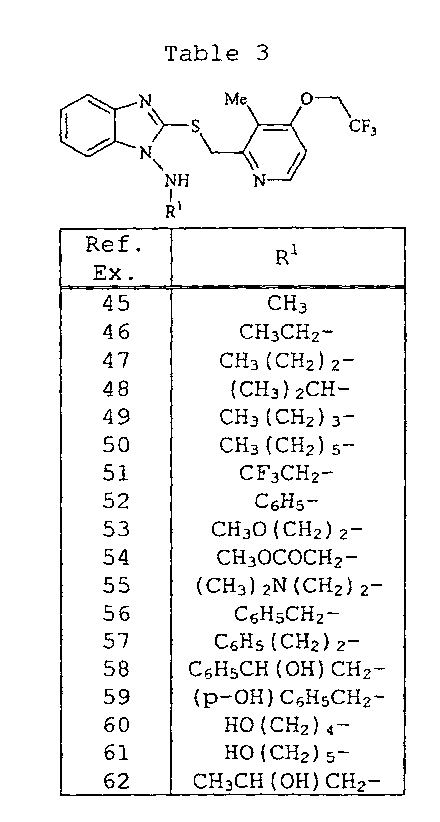

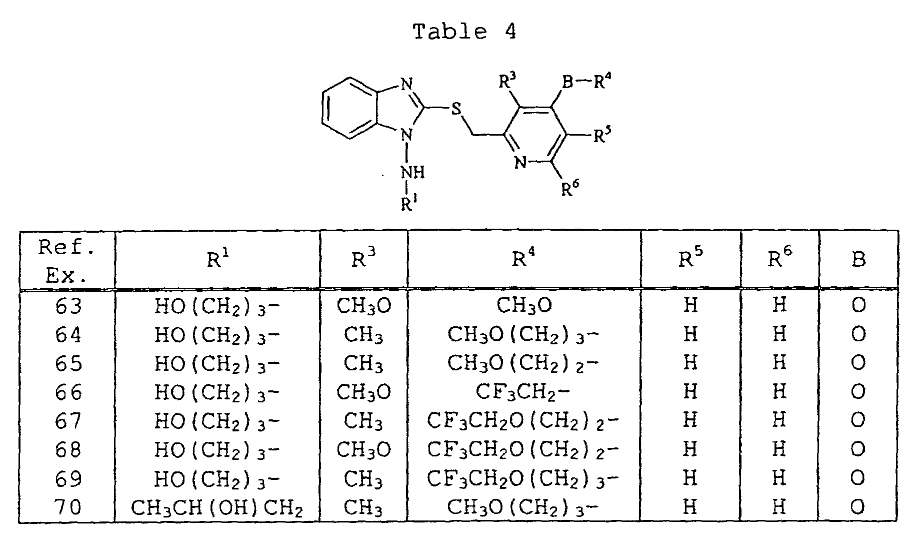

### Physicochemical Data

### Referential Example 45

¹H-NMR(CDCl₃)δ:
2.35(3H,s)2.95(3H,d),4.39(2H,q),4.79(2H,s),4.82(1H,q),6.65(1H,d),7.19-7.36(3H,m),7.66-7.70(1H,m),8.37(1H,d);
IR(KBr,cm⁻¹): 3221,1578,1475,1259,1161; MS (FAB,MH⁺): 383;
Melting point: 113-114°C

### Referential Example 46

¹H-NMR(DMSO-d₆)δ:
1.01(3H,t),2.26(3H,s),3.05-3.15(2H,m),4.68(2H,s),4.89(2H,q),6.72(1H,t),7.09(1H,d),7.13-7.56(4H,m),8.31(1H,d);
IR(KBr,cm⁻¹): 3400, 3212, 1586; MS(EI,M⁺): 396

### Referential Example 47

¹H-NMR(CDCl₃)δ:
0.98(3H,t),1.51-1.65(2H,m),2.35(3H,s),3.14-3.21(2H,m),4.40(2H,q),4.79(2H,s),4.82(1H,t),6.65(1H,d),7.18-7.36(3H,m),7.66-7.71(1H,m),8.37(1H,d); IR(KBr,cm⁻¹):
3206,1578,1458,1309,1271,1159,980; MS(EI,M⁺): 410; Melting pint: 112-113°C

### Referential Example 48

¹H-NMR(CDCl₃)δ:
1.10(6H,d),2.35(3H,s),3.68-3.73(1H,m),4.39(2H,q),4.76-4.79(1H,m),4.79(2H,s),6.65(1H,d),7.17-7.35(3H,m),7.65-7.69(1H,m),8.37(1H,d); IR(KBr,cm⁻¹): 3225,1581,1427,1257,1163;
MS(EI,M⁺): 410; Melting point: 131-133°C

### Referential Example 49

¹H-NMR(CDCl₃)δ:
0.92(3H,t)1.35-1.59(4H,m),2.35(3H,s)3.17-3.24(2H,m),4.39(2H,q),4.79(2H,s),4.80(1H,t),6.65(1H,d),7.18-7.35(3H,m),7.64-7.71(1H,m),8.37(1H,d); IR(KBr,cm⁻¹):
3242,1587,1437,1313,1263,1169,1113; MS (EI,M⁺): 424; Melting point: 83-84°C

### Referential Example 50

¹H-NMR(CDCl₃)δ:
0.87(3H,t),1.27-1.58(8H,m),2.35(3H,s),3.19(2H,q),4.39(2H,q),4.79(2H,s),4.80(1H,t),6.65(1H,d),7.19-7.35(3H,m),7.66-7.69(1H,m),8.37(1H,d); IR(KBr,cm⁻¹):
3192,1581,1360,1272,1165; MS(EI,M⁺): 452; Melting point: 72-73°C

### Referential Example 51

¹H-NMR(CDCl₃)δ:
2.33(3H,s),3.72-3.83(2H,m),4.39(2H,q),4.72(2H,s),5.83(1H,bs),6.65(1H,d),7.20-7.68(4H,m),8.34(1H,d); MS(EI,M⁺): 450

### Referential Example 52

¹H-NMR(DMSO-d₆)δ:
2.25(3H,s),4.75(2H,s),4.88(2H,q),6.47(2H,d),6.84(1H,t),7.07-7.21(6H,m),7.62(1H,d),8.27(1H,d),9.45(1H,s);
IR(KBr,cm⁻¹): 3167,1603,1583; MS(EI,M⁺): 444; Melting point: 184-186°C

### Referential Example 53

¹H-NMR(CDCl₃)δ:
2.36(3H,s),3.32-3.38(2H,m),3.42(3H,s),3.54-3.58(2H,m),4.39(2H,q),4.79(2H,s),5.18(1H,t),6.65(1H,d),7.18-7.40(3H,m),7.66-7.69(1H,m),8.37(1H,d)

### Referential Example 54

¹H-NMR(CDCl₃)δ:
2.35(3H,s),3.79(3H,s),3.95(2H,d),4.39(2H,q),4.77(2H,s),5.11(1H,d),6.66(1H,d),7.21-7.41(3H,m),7.65-7.68(1H,m),8.37(1H,d); IR(KBr,cm⁻¹): 3244,1747,1587,1439,1269,1163,1115;
MS(FAB,MH⁺): 441; Melting point: 154-156°C

### Referential Example 55

¹H-NMR(CDCl₃)δ:
2.30(6H,s),2.35(3H,s),2.52-2.56(2H,m),3.18-3.24(2H,m),4.39(2H,q),4.79(2H,s),5.30(1H,t),6.65(1H,d),7.20-7.37(3H,m),7.66-7.69(1H,m),8.37(1H,d)

### Referential Example 56

¹H-NMR(CDCl₃)δ:
2.33(3H,s),4.30(2H,d),4.39(2H,q),4.77(2H,s),5.08(1H,t),6.65(1H,d),7.19-7.42(8H,m),7.65-7.69(1H,m),8.35(1H,d)

### Referential Example 57

¹H-NMR(CDCl₃)δ:
2.33(3H,s),2.90(2H,t),3.46(2H,q),4.39(2H,q),4.78(2H,s),4.85(1H,t),6.65(1H,d),7.12-7.37(8H,m),7.64-7.68(1H,m),8.36(1H,d)

### Referential Example 58

¹H-NMR(CDCl₃)δ:
2.35(3H,s),3.31-3.47(3H,m),4.38(2H,q),4.79(2H,s),4.80-4.85(1H,m),5.34(1H,t),6.62(1H,d),7.20-7.37(8H,m),7.65-7.68(1H,m),8.30(1H,d)

### Referential Example 59

¹H-NMR(DMSO-d₆)δ:
2.25(3H,s),4.06(2H,d),4.64(2H,s),4.89(2H,q),6.66-6.69(2H,m),6.94(1H,t),7.08-7.15(5H,m),7.43-7.54(2H,m),8.32(1H,d),9.31(1H,s)

### Referential Example 60

¹H-NMR(CDCl₃)δ:
1.62-1.90(5H,m),2.35(3H,s),3.22-3.29(2H,m),3.65-3.67(2H,m),4.40(2H,q),4.77(2H,s),4.97(1H,t),6.65(2H,d),7.29-7.36(3H,m),7.65-7.69(1H,m),8.36(1H,d)

### Referential Example 61

¹H-NMR(CDCl₃)δ:
1.45-1.70(7H,m),2.36(3H,s),3.20-3.27(2H,m),3.60-3.66(2H,m),4.40(2H,q),4.78(2H,s),4.85(1H,t),6.66(1H,d),7.20-7.35(3H,m),7.66-7.69(1H,m),8.36(1H,d)

### Referential Example 62

¹H-NMR(CDCl₃)δ:
1.17(3H,d),2.35(3H,s),2.91(1H,d),3.03(1H,dq),3.33(1H,dq),3.82-3.96(1H,m),4.40(2H,q),4.79(2H,s),5.22(1H,t),6.65(1H,d),7.18-7.30(2H,m),7.32-7.40(1H,m),7.63-7.71(1H,m),8.35(1H,d); IR(KBr,cm⁻¹):
3247,3050,2965,2926,1592,1576,1474,1456,1424,1308,1275,1254,1177,1121,974,789; MS(FAB,MH⁺): 427; Melting point: 143-144°C

### Referential Example 63

¹H-NMR(CDCl₃)δ:
1.79-1.83(2H,m),2.50-2.58(1H,m),3.34-3.36(2H,m),3.90-3.92(2H,m),3.91(6H,s),4.76(2H,s),5.34(1H,t),6.78(1H,d),7.19-7.37(3H,m),7.65-7.67(1H,m),8.22(1H,d)

### Referential Example 64

¹H-NMR(CDCl₃)δ:
1.80-1.84(2H,m),2.06-2.11(2H,m),2.29(3H,s),2.55-2.60(1H,m),3.33-3.41(2H,m),3.37(3H,s),3.54-3.59(2H,m),3.87-3.88(2H,m),4.08-4.13(2H,m),4.73(2H,s),5.31(1H,t),6.70(1H,d),7.20-7.38(3H,m),7.66-7.67(1H,m),8.29(1H,d)

### Referential Example 65

¹H-NMR(CDCl₃)δ:
1.79-1.83(2H,m),2.32(3H,s),2.71-2.80(1H,m),3.34-3.36(2H,m),3.46(3H,s),3.77-3.90(4H,m),4.15-4.17(2H,m),4.73(2H,s),5.31(1H,t),6.69(1H,d),7.19-7.36(3H,m),7.65-7.69(1H,m),8.29(1H,d)

### Referential Example 66

¹H-NMR(CDCl₃)δ:
1.78-1.83(2H,m),2.42(1H,t),3.32-3.38(2H,m),3.86-3.90(2H,m),3.96(3H,s),4.43(2H,q),4.78(2H,s),5.26(1H,t),6.75(1H,d),7.19-7.37(3H,m),7.65-7.68(1H,m),8.23(1H,d)

### Referential Example 67

¹H-NMR(CDCl₃)δ:
1.80-1.84(2H,m),2.31(3H,s),2.35-2.40(1H,m),3.35-3.38(2H,m),3.89-4.05(4H,m),3.96(2H,q),4.17-4.21(2H,m),4.74(2H,s),5.27(1H,t),6.69(1H,d),7.21-7.38(3H,m),7.60-7.68(1H,m),8.31(1H,d)

### Referential Example 68

¹H-NMR(CDCl₃)δ:
1.78-1.83(2H,m),2.49(1H,t),3.34-3.36(2H,m),3.88-4.06(6H,m),3.97(3H,s),4.21-4.25(2H,m),4.76(2H,s),5.32(1H,t),6.77(1H,d),7.19-7.35(3H,m),7.60-7.70(1H,m),8.19(1H,d)

### Referential Example 69

¹H-NMR(CDCl₃)δ:
1.80-1.84(2H,m),2.10-2.15(2H,m),2.29(3H,s),2.50-2.57(1H,m),3.35-3.40(2H,m),3.78-3.91(6H,m),4.10-4.14(2H,m),4.73(2H,s),5.30(1H,t),6.70(1H,d),7.20-7.38(3H,m),7.66-7.69(1H,m),8.30(1H,d)

### Referential Example 70

¹H-NMR(CDCl₃)δ:
1.17(3H,d),2.04-2.13(2H,m),2.29(3H,s),2.91(1H,d),3.02(1H,dq),3.33(1H,dq),3.36(3H,s),3.54-3.59(2H,m),3.82-3.96(1H,m),4.08-4.14(2H,m),4.76(2H,s),5.25(1H,t),6.70(1H,d),7.21-7.26(2H,m),7.35-7.38(1H,m),7.66-7.69(1H,m),8.29(1H,d)

### Referential Example 71

### Synthesis of 1-amino-5-hydroxymethyl-2-[[4-(2,2,2-trifluoroethoxy)-3-methylpyridin-2-yl]methylthio]benzimidazole and 1-amino-6-hydroxymethyl-2-[[4-(2,2,2-trifluoroethoxy)-3-methylpyridin-2-yl]methylthio]benzimidazole

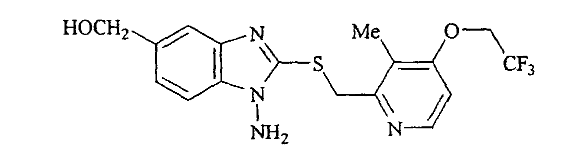

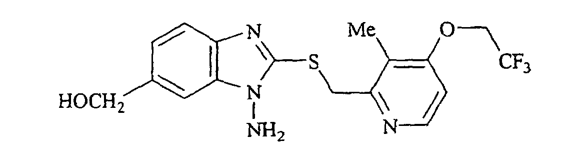

### Step 1

### Synthesis of 5-hydroxymethyl-2-mercaptobenzimidazole

Lithium aluminum hydride (2.1 g) was suspended in anhydrous tetrahydrofuran (100 mL), followed by the addition of 2-mercapto-5-methoxycarbonylbenzimidazole (4.8 g) under an argon atmosphere at 10°C. The resulting mixture was stirred at room temperature for 1 hour. Water was added to the react ion mixture, and the mixture was stirred for 10 minutes. The thus-obtained mixture was filtered through celite, and the filtrate was concentrated under reduced pressure. 1N Hydrochloric acid was added to the residue to adjust its pH to 4. The precipitated crystals were collected by filtration and then dried to afford the title compound (2.85 g).
¹H-NMR(DMSO-d₆)δ:
4.52(1H,t),4.64(2H,d),7.11(2H,s),7.22(1H,s),7.53(1H,s),12.20(1H,bs)

### Step 2

### Synthesis of 5-hydroxymethyl-2-[[4-(2,2,2-trifluoroethoxy)-3-methylpyridin-2-yl]methylthio]-1H-benzimidazole

5-Hydroxymethyl-2-mercaptobenzimidazole (2.8 g) was suspended in methanol (40 mL), followed by the addition of 2-chloromethyl-3-methyl-4-(2,2,2-trifluoroethoxy)pyridine hydrochloride (4.3 g) and sodium hydroxide (1.4 g). The resulting mixture was stirred at 50°C for 1 hour. Water was added to the reaction mixture, and extraction was conducted with ethyl acetate. The organic layer was successively washed with a saturated aqueous solution of sodium bicarbonate, water and brine, and then dried over anhydrous sodium sulfate. The solvent was distilled off under reduced pressure. The resultant crystals were washed with isopropyl ether, and then dried to afford the title compound (5.8 g).
¹H-NMR (CDCl₃)δ:
2.33(3H,s),3.82-4.10(1H,m),4.48(2H,q),4.58(2H,s),4.74(2H,d),6.78(1H,d),7.20-7.60(3H,m),8.39(1H,d),12.45(1H,bs)
Melting point: 153-155°C

### Step 3

### Synthesis of 1-(N-tert-butyloxycarbonylamino)-5-hydroxymethyl-2-[[4-(2,2,2-trifluoroethoxy)-3-methylpyridin-2-yl]methylthio]benzimidazole and 1-(N-tert-butyloxycarbonylamino)-6-hydroxymethyl-2-[[4-(2,2,2-trifluoroethoxy)-3-methylpyridin-2-yl]methylthio]benzimidazole

5-Hydroxymethyl-2-[[4-(2,2,2-trifluoroethoxy-3-methylpyridin-2-yl)methylthio]-1H-benzimidazole (3.4 g) was dissolved in N,N-dimethylformamide (20 mL). Under ice cooling, potassium carbonate (1.28 g) and N-tert-butoxycarbonyl-3-(4-cyanophenyl)oxaziridine (2.2 g), which had been obtained by a known process (J. Org. Chem., 58, 4791 (1993); Tetrahedronlett., 36, 1439 (1995)), were added. The resulting mixture was stirred at the same temperature for 1 hour. Water was added to the reaction mixture, and extraction was conducted with ethyl acetate. The organic layer was successively washed with water and brine, and then dried over anhydrous sodium sulfate. The solvent was distilled off under reduced pressure. The residue was purified by chromatography on a silica gel column (silica gel: "NH-DM1020" (product of FUJI SILYSIA CHEMICAL LTD.), hexane:ethyl acetate = 1:1) to afford the title compound (5.8 g; a 1:1 mixture of the 5-hydroxymethyl isomer and the 6-hydroxymethyl isomer).
¹H-NMR(CDCl₃)δ:
1.47(9H,bs),2.33(3H,s),2.34-2.60(1H,m),4.39(2H,q),4.65(1H,bs),4.69(2H,s),4.77(1H,bs),6.65(1H,d),7.13-7.53(3H,m),8.32(1H,d),8.50(1H,bs)

### Step 4

### Synthesis of 1-amino-5-hydroxymethyl-2-[[4-(2,2,2-trifluoroethoxy)-3-methylpyridin-2-yl]methylthio]benzimidazole and 1-amino-6-hydroxymethyl-2-[[4-(2,2,2-trifluoroethoxy)-3-methylpyridin-2-yl]methylthio]benzimidazole

The mixture (3.5 g) of 1-(N-tert-butyloxycarbonylamino)-5-hydroxymethyl-2-[[4-(2,2,2-trifluoroethoxy)-3-methylpyridin-2-yl]methylthio]benzimidazole and 1-(N-tert-butyloxycarbonylamino)-6-hydroxymethyl-2-[[4-(2,2,2-trifluoroethoxy)-3-methylpyridin-2-yl]methylthio]benzimidazole, which had been obtained in Step 3, was dissolved in a mixed solvent of ethanol (12 mL) and dioxane (12 mL), followed by the addition of a 4N solution of hydrochloric acid in dioxane (24 mL). The resulting mixture was stirred at 50°C for 2 hours. The reaction mixture was concentrated under reduced pressure, a saturated aqueous solution of sodium bicarbonate was added to the residue, and extraction was conducted with ethyl acetate. The organic layer was successively washed with water and brine, and then dried over anhydrous sodium sulfate. The solvent was distilled off under reduced pressure. The residue was purified by chromatography on a silica gel column (silica gel: "NH-DM1020" (product of FUJI SILYSIA CHEMICAL LTD.), chloroform:methanol = 10:1). Recrystallization was then conducted from a mixed solvent of hexane and ethyl acetate to afford the title compound (1.92 g; a 1:1 mixture of the 5-hydroxymethyl isomer and the 6-hydroxymethyl isomer).
¹H-NMR(DMSO-d₆)δ:
2.27(3H,s),4.56(1H,d),4.60(1H,d),4.67(2H,s),4.90(2H,q),5.10(0.5H,t),5.20(0.5H,t),5.98(2H,s),7.06-7.45(3H,m),7.09(1H,d),8.31(1H,d)
IR(KBr,cm⁻¹): 3300,1578,1439,1309,1255,1170,1109,1018
MS(EI,M⁺): 398

The following compounds were then obtained choosing by raw materials as needed and conducting a similar procedure as in Referential Example 71.

### Referential Example 72

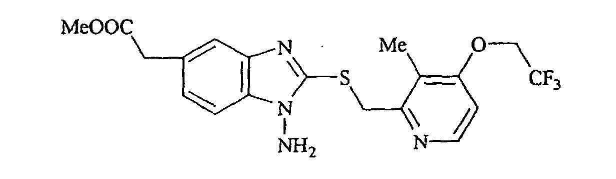

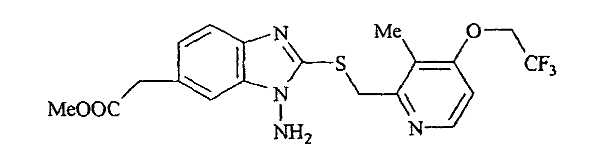

¹H-NMR(CDCl₃)δ:
2,35(3H,s),3.65(1.5H,s),3.67(1.5H,s),3.74(1H,s),3.77(1H,s),4.39(2H,q),4.72(2H,s),4.79(2H,s),6.65(1H,d),7.10-7.32(2H,m),7.55-7.60(1H,m),8.34(1H,d)

### Referential Example 73

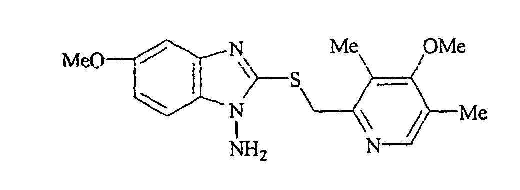

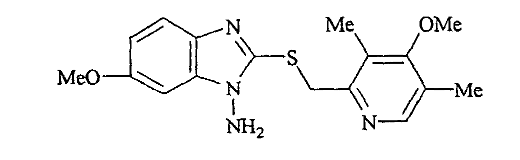

¹H-NMR(DMSO-d₆)δ:
2.21(3H,s),2.29(1.5H,s),2.30(1.5H,s),3.73(2.5H,s),3.74(1.5H,s),3.77(1.5H,s),3.79(1.5H,s),4.59(1H,s),4.62(1H,s),5.91(1H,s),5.93(1H,s),6.72-6.93(1.5H,m),7.07-7.40(1.5H,m),8.18(1H,s)

### Referential Example 74

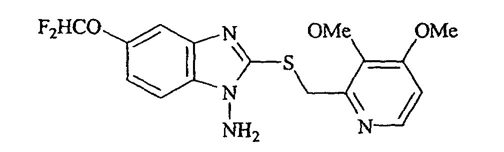

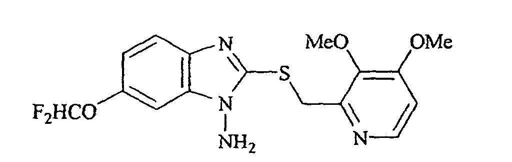

¹H-NMR(CDCl₃)δ:
3.91(3H,s),3.92(3H,s),4.73(1H,s),4.75(1H,s),4.79(1H,s),4.82(1H,s),6.49(0.5H,t),6.52(0.5H,t),6.79(1H,d),6.99(0.5H,dd),7.04(0.5H,dd),7.18(0.5H,d),7.33(0.5H,d),7.42(0.5H,d),7.58(0.5H,d),8.19(1H,d)

### Referential Example 75

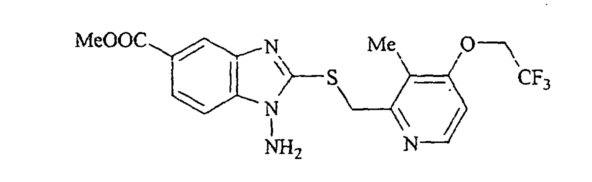

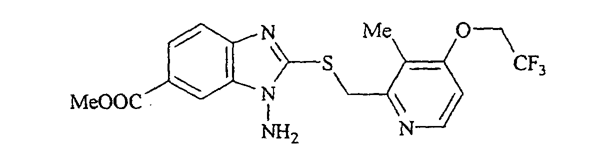

¹H-NMR(CDCl₃)δ:
2.37(3H,s),3.94(1.5H,s),3.95(1.5H,s),4.40(2H,q),4.75(1H,s),4.76(1H,s),4.79(1H,s),4.81(1H,s),6.66(1H,d),7.39(0.5H,d),7.64(0.5H,d),7.93-8.00(1H,m),8.09(0.5H,d),8.34-8.37(1.5H,m)
IR(KBr,cm⁻¹): 3352,1707,1579,1429,1304,1170,1111,983

### Referential Example 76

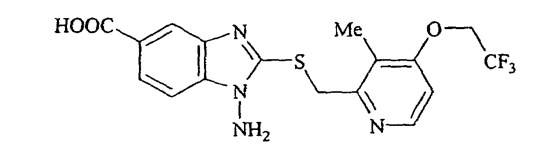

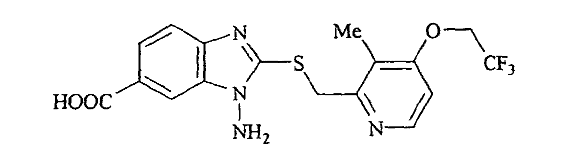

¹H-NMR(DMSO-d₆)δ:
2.28(3H,s),4.71(2H,s),4.90(2H,q),6.13(1H,s),6.15(1H,s),7.10(1H,d),7.43(0.5H,d),7.55(0.5H,d),7.77(0.5H,dd),7.82(0.5H,dd),8.03(0.5H,d),8.06(0.5H,d),8.32(1H,d),12.70(1H,bs)
IR(KBr,cm⁻¹) :3300,1684,1581,1431,1257,1169,1113,974

### Referential Example 77

### Synthesis of 1-amino-5-difluoromethoxy-2-[[4-(2,2,2-trifluoroethoxy)-3-methylpyridin-2-yl]methylthio]benzimidazole

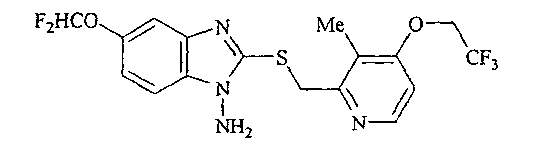

### Step 1

### Synthesis of 5-difluoromethoxy-2-hydrazinonitrobenzene

2-Amino-5-fluoromethoxynitrobenzene (2.9 g) was suspended in conc. hydrochloric acid (30 mL), followed by the dropwise addition at -5°C of an aqueous solution of sodium nitrite (1.03 g) in water (30 mL). The resulting mixture was stirred at the same temperature for 1 hour. A solution of tin dichloride dihydrate (6.6 g) in conc. hydrochloric acid (18 mL) was then added, followed by stirring at the same temperature for 2 hours. The reaction mixture was poured into ice water, the water layer was washed with chloroform, and subsequent to neutralization with sodium carbonate, extraction was conducted with ethyl acetate. The organic layer was successively washed with water and brine and dried over anhydrous sodium sulfate, and subsequently, the solvent was distilled off to afford the title compound (1.6 g).

### Step 2

### Synthesis of 2-(2-acetylhydrazino)-5-difluoromethoxynitrobenzene

5-Difluoromethoxy-2-hydrazinonitrobenzene (1.6g) was dissolved in chloroform (15 mL), followed by the addition of acetic anhydride (5 mL) and pyridine (0.1 mL). The resulting mixture was stirred at room temperature for 2 hours. The reaction mixture was concentrated under reduced pressure, a saturated aqueous solution of sodium bicarbonate was added to the residue to alkalinize the same, and extraction was conducted with ethyl acetate. The organic layer was successively washed with water and brine, and then dried over anhydrous sodium sulfate. The solvent was distilled off under reduced pressure. The residue was purified by chromatography on a silica gel column (silica gel: "NH-DM1020" (product of FUJI SILYSIACHEMICALLTD.), ethyl acetate) to afford the title compound (1.3 g).
¹H-NMR(DMSO-d₆)δ:
3.32(3H,s),7.14(1H,d),7.19(1H,t),7.48(1H,dd),7.87(1H,d),9.22(1H,s),10.14(1H,s)

### Step 3

### Synthesis of 1-acetylamino-5-difluoromethoxy-2-mercaptobenzimidazole

2-(2-Acetylhydrazino)-5-difluoromethoxynitrobenzene (1.6 g) was dissolved in tetrahydrofuran (20 mL), followed by the addition of 10% palladium-charcoal (160 mg). The resulting mixture was stirred under a hydrogen atmosphere at room temperature for 2.5 hours. The reaction mixture was filtered through celite and then concentrated under reduced pressure. The reaction mixture was added to a mixture which had been obtained by combining ethanol (10 mL), water (10 mL), carbon disulfide (5 mL) and potassium hydroxide (444 mg) and then stirring them at room temperature for 30 minutes. The resulting mixture was heated under reflux for 3 hours. 1N Hydrochloric acid was added to the reaction mixture to acidify the same, and then, extraction was conducted with ethyl acetate. The organic layer was successively washed with water and brine, and then dried over anhydrous sodium sulfate. The solvent was distilled off under reduced pressure, and the resultant crystals were recrystallized from a mixed solvent of isopropyl ether and hexane to afford the title compound (1.6 g).
¹H-NMR(DMSO-d₆)δ:
3.30(3H,s),6.99-7.16(3H,m),7.20(1H,t),11.19(1H,s),13.10(1H,s)

### Step 4

### Synthesis of 1-amino-5-difluoromethoxy-2-mercaptobenzimidazole monohydrochloride

1-Acetylamino-5-difluoromethoxy-2-mercaptobenzimidazole (1.53 g) was dissolved in ethanol (20 mL), followed by the addition of conc. hydrochloric acid (4 mL) and water (20 mL). The resulting mixture was heated under reflux for 4 hours. The reaction mixture was concentrated under reduced pressure. The concentrate was subjected along with ethanol to azeotropic distillation. The resultant crystals were washed with isopropyl ether, and then dried to afford the title compound (1.06 g).

### Step 5

### Synthesis of 1-amino-5-difluoromethoxy-2-[[4-(2,2,2-trifluoroethoxy)-3-methylpyridin-2-yl]methylthio]benzimidazole

1-Amino-5-difluoromethoxy-2-mercaptobenzimidazole monohydrochloride (800 mg) was suspended in ethanol (20 mL), followed by the addition of sodium hydroxide (418 mg). The resulting mixture was stirred at 50°C for 5 minutes, 2-chloromethyl-3-methyl-4-(2,2,2-trifluoroethoxy)pyridine hydrochloride (995 mg) was added, and the resulting mixture was stirred at the same temperature for 1.5 hours. Water was added to the reaction mixture, and extraction was conducted with ethyl acetate. The organic layer was successively washed with water and brine, and then dried over anhydrous sodium sulfate. The solvent was distilled off under reduced pressure. The residue was purified by chromatography on a silica gel column (silica gel: "NH-DM1020" (product of FUJI SILYSIA CHEMICAL LTD.), chloroform:methanol = 10:1).
Recrystallization was then conducted from a mixed solvent of n-hexane and ethyl acetate to afford the title compound (1.26 g).
¹H-NMR(DMSO-d₆)δ:
2.27(3H,s),4.68(2H,s),4.90(2H,q),6.60(2H,s),7.02(1H,dd),7.09(1H,d),7.15(1H,t),7.33(1H,d),7.37(1H,d),8.32(1H,d)
IR(KBr,cm⁻¹): 1578,1402,1379,1259,1184,1113,1047
MS(FAB,MH⁺): 435
Melting point: 179-180°C

The following compounds were then obtained by choosing raw materials as needed and conducting a similar procedure as in Referential Example 77.

### Referential Example 78

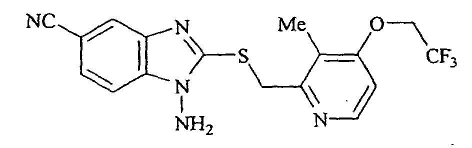

¹H-NMR(DMSO-d₆)δ:
2.27(3H,s),4.71(2H,s),4.90(2H,q),6.19(2H,bs),7.10(1H,d),7.52(1H,d),7.59(1H,dd),8.03(1H,d),8.32(1H,d)
IR(KBr,cm⁻¹): 3332, 2224, 1583

### Referential Example 79

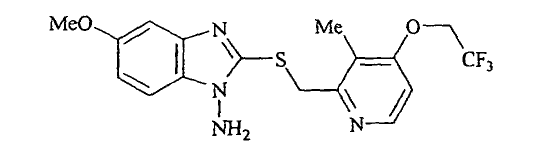

¹H-NMR(DMSO-d₆)δ:
2.26(3H,s),3.77(3H,s),4.66(2H,s),4.89(2H,q),5.94(2H,s),6.79(1H,dd),7.08(1H,d),7.09(1H,d),7.24(1H,d),8.31(1H,d)
IR(KBr,cm⁻¹): 3312,3134,1649,1620,1581
MS(EI,M⁺): 396
Melting point: 178-180°C

### Referential Example 80

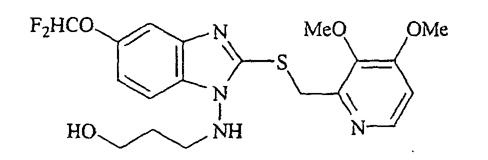

¹H-NMR(CDCl₃)δ:
1.75-1.80(2H,m),3.28-3.35(3H,m),3.83-3.87(2H,m),3.91(6H,s),4.73(2H,s),5.44(1H,t),6.48(1H,t),6.78(1H,d),6.95(1H,dd),7.27(1H,d),7.39(1H,d),8.18(1H,d)

### Referential Example 81

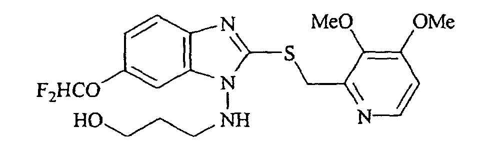

¹H-NMR(CDCl₃)δ:
1.78-1.86(2H,m),2.43(1H,t),3.26-3.36(2H,m),3.83-3.91(2H,m),3.91(6H,s),4.73(2H,s),5.39(1H,t),6.52(1H,t),6.80(1H,d),6.99(1H,dd),7.16(1H,d),7.60(1H,d),8.21(1H,d)

### Referential Example 82

### Synthesis of 1-amino-2-[[4-(2,2,2-trifluoroethoxy)-3-methylpyridin-2-yl]methylthio]pyrido(2,3-d)imidazole

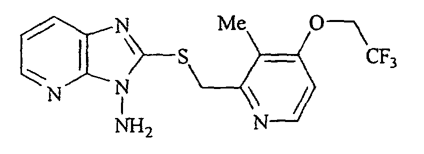

### Step 1

### Synthesis of 2-(2-tert-butyloxycarbonylhydrazino)-3-nitropyridine

To a suspension of 2-chloro-3-nitropyridine (5.0 g) in 95% ethanol (40 mL), a solution of hydrazine monohydrate (4.8 g) in 95% ethanol (10 mL) was added dropwise under ice cooling. The resulting mixture was stirred at 25 to 30°C for 1 hour. Isopropyl ether was added to the reaction mixture, and the precipitated crystals were collected by filtration and dried to yield 2-hydrazino-3-nitropyridine. It was dissolved in 1,4-dioxane, followed by the addition of di(tert-butyl) dicarbonate (17.6 g) , water (10 mL) and potassium carbonate (11.2 g). The resulting mixture was stirred at room temperature for 12 hours. Water was added to the reaction mixture, and extraction was conducted with ethyl acetate. The organic layer was successively washed with water and brine, and then dried over anhydrous sodium sulfate. The solvent was distilled off under reduced pressure, and the resultant crystals were recrystallized from isopropyl ether to afford the title compound (5.1 g).
¹H-NMR(DMSO-d₆)δ:
1.43(9H,s),6.95(1H,dd),8.47(1H,dd),8.50-8.53(1H,m),9.05(1H,s),9.57(1H,s)

### Step 2

### Synthesis of 1-(N-tert-butyloxycarbonylamino)-2-mercaptopyrido(2,3-d)imidazole

2-(2-tert-Butyloxycarbonylhydrazino)-3-nitropyridine (3.3 g) was dissolved in a mixed solvent of ethanol (30 mL) and tetrahydrofuran (5 mL), followed by the addition of 10% palladium-charcoal (50% water content; 660 mg). The resulting mixture was stirred under a hydrogen atmosphere at room temperature for 3 hours. The reaction mixture was filtered through celite, and the filtrate was concentrated under reduced pressure. The reaction mixture was added to a mixture which had been obtained by combining ethanol (10 mL), water (20 mL), carbon disulfide (10 mL) and potassium hydroxide (900 mg) and then stirring them at room temperature for 30 minutes. The resulting mixture was heated under reflux for 2 hours. Acetic acid was added to the reaction mixture, and then, extraction was conducted with ethyl acetate. The organic layer was successively washed with water and brine, and then dried over anhydrous sodium sulfate. The solvent was distilled off under reduced pressure, and the residue was added to isopropyl ether to crystallize the same. The resultant crystals were collectedby filtration and then dried to afford the title compound (2.9 g).
¹H-NMR(CDCl₃)δ:
1.54(9H,s),7.11(1H,dd),7.43(1H,dd),8.19(1H,dd),8.37(1H,s),12.76(1H,s)

### Step 3

### Synthesis of 1-amino-2-[[4-(2,2,2-trifluoroethoxy)-3-methylpyridin-2-yl]methylthio]pyrido(2,3-d)imidazole

The title compound was obtained by conducting a similar procedure as in Step 3 of Referential Example 1 and then repeating the procedure of Step 4.
¹H-NMR(DMSO-d₆)δ:
2.27(3H,s),4.70(2H,s),4.91(2H,q),6.02(2H,s),7.10(1H,d),7.21(1H,dd),7.91(1H,dd),8.22(1H,dd),8.33(1H,d)
IR (KBr, cm⁻¹): 1579, 1456, 1390, 1259, 1147, 1113
MS(FAB,MH⁺): 370
Melting point: 194-195°C

The following compounds were then obtained by choosing raw materials as needed and conducting a similar procedure as in Referential Example 82.

### Referential Example 83

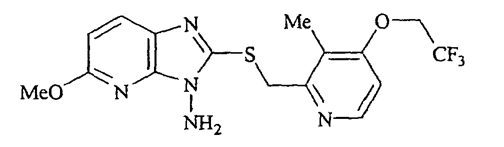

¹H-NMR(DMSO-d₆)δ:
2.25(3H,s),3.92(3H,s),4.64(2H,s),4.90(2H,q),5.93(2H,s),6.62(1H,d),7.09(1H,d),7.84(1H,d),8.32(1H,d)
IR(KBr,cm⁻¹): 1593,1448,1373,1255,1161,1101,1032
MS(FAB,MH⁺): 400
Melting point: 192-194°C

### Referential Example 84

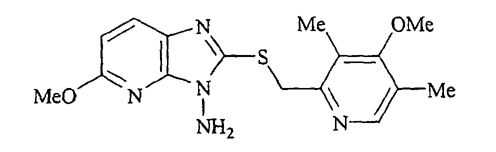

¹H-NMR(CDCl₃)δ:
2.24(3H,s),2.35(3H,s),3.39(3H,s),3.76(3H,s),4.68(2H,s),4.78(2H,s),6.68(2H,s),4.78(2H,s),6.68(1H,d),7.79(1H,d),8.20(1H,s)

### Referential Example 85

### Synthesis of 1-amino-2-[[4-(2,2,2-trifluoroethoxy)-3-methylpyridin-2-yl]methylthio]thieno(3,4-d)imidazole

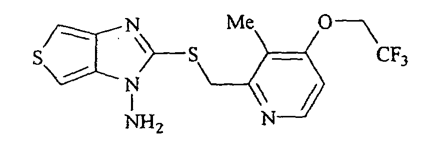

### Step 1

### Synthesis of 2,5-dibromo-3,4-dinitrothiophene

2,5-Dibromothiophene (38.0 g) was added dropwise at 0°C to conc. sulfuric acid (240 mL), followed by the dropwise addition of fuming nitric acid (50 mL). The resulting mixture was stirred at the same temperature for 2 hours. The reaction mixture as poured into ice water. The precipitated crystals were collectedby filtration, and was then recrystallized from methanol to afford the title compound (20.3 g).

### Step 2

### Synthesis of 2-mercaptothieno(3,4-d)imidazole

2, 5-Dibromo-3,4-dinitrothiophene (20.2 g) was added to conc. hydrochloric acid (300 mL), followed by the addition of metal tin (40 g) at 20°C. The resultingmixture was stirred at room temperature for 12 hours. The reaction mixture was filtered, and the filtrate was concentrated under reduced pressure. The precipitated crystals were collected by filtration and then washed with diethyl ether. The crystals were suspended in ethyl acetate, followed by the addition of a 25% aqueous solution of sodium hydroxide. The resulting mixture was filtered through celite, and the filtrate was extracted with ethyl acetate. The organic layer was washed with brine, and then dried over anhydrous sodium sulfate. The solvent was distilled off to yield 3, 4-diaminothiophene (3. 9 g). It was added to a mixture which had been obtained by combining ethanol (84 mL), water (84 mL), carbon disulfide (15 mL) and potassium hydroxide (2. 4 g) and then stirring them at room temperature for 30 minutes. The resulting mixture was stirred at 60°C for 3 hours. Acetic acid and water were added to the reaction mixture, and then, extraction was conducted with ethyl acetate. The organic layer was successively washed with water and brine, and then dried over anhydrous magnesium sulfate. The solvent was distilled off under reduced pressure. The resultant crystals were washed with a mixed solvent of ethanol and chloroform, collected by filtration, and then dried to afford the title compound (2.0 g).
¹H-NMR(DMSO-d₆)δ: 6.70(2H,s),12.0(2H,s)

### Step 3

### Synthesis of 1-amino-2-[[4-(2,2,2-trifluoroethoxy)-3-methylpyridin-2-yl]methylthio]thieno(3,4-d)imidazole

The title compound was obtained by conducting a similar procedure as in Step 3 of Referential Example 1 and then repeating the procedure of Step 4 of Referential Example 1. ¹H-NMR(DMSO-d₆)δ:
2.25(3H,s),4.59(2H,s),4.91(2H,q),5.82(2H,s),6.67(1H,d),7.01(1H,d),7.09(1H,d),8.32(1H,d)
IR(KBr,cm⁻¹): 3324, 3065, 1579, 1458, 1257, 1115
MS(FAB,MH⁺): 375
Melting point: 163-166°C (decomposed)

The following compounds were then obtained by choosing raw materials as needed and conducting a procedure as in Referential Example 85.

### Referential Example 86

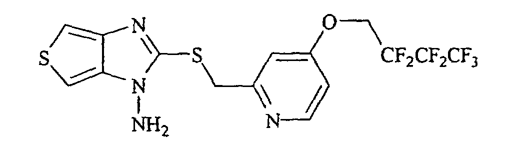

¹H-NMR(DMSO-d₆)δ:
4.50(2H,s),4.99(2H,t),5.82(2H,s),6.67(1H,d),6.99(1H,d),7.05(1H,dd),7.28(1H,d),8.42(1H,d)
IR (KBr, cm⁻¹):
3071,1595,1437,1313,1252,1124,960,920,882,762,730,656
MS(FAB,MH⁺): 461
Melting point: 152-154°C (decomposed)

### Referential Example 87

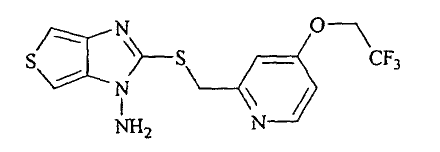

¹H-NMR(DMSO-d₆)δ:
4.51(2H,s),4.97(2H,t),5.80(2H,s),6.69(1H,d),7.01(1H,d),7.08(1H,dd),7.31(1H,d),8.44(1H,d)
IR(KBr,cm⁻¹):
3333,1599,1437,1329,1309,1215,1113,958,862,806,746,679,572
MS(FAB,MH⁺): 361
Melting point: 121-122°C (decomposed)

### Example 1

### Synthesis of 1-amino-2-[[4-(2,2,2-trifluoroethoxy)-3-methylpyridin-2-yl]methylsulfinyl]benzimidazole

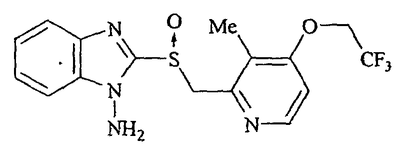

1-Amino-2-[[4-(2,2,2-trifluoroethoxy)-3-methylpyridin-2-yl]methylthio]benzimidazole (33.9 g) was suspended in methylene chloride (400mL), followed by the dropwise addition at -20°C of a solution of 80% m-chloroperbenzoic acid (19.9 g) in methylene chloride (300 mL). The resulting mixture was stirred at the same temperature for 1 hour. To the reaction mixture, anaqueous solution (200mL) with sodium sulfite (15.6 g) dissolved therein was added dropwise. The temperature of the resulting mixture was allowed to rise to room temperature, followed by the addition of a saturated aqueous solution of sodium bicarbonate. The thus-obtained mixture was stirred for 10 minutes, and then extracted with chloroform. The organic layer was successively washed with water and brine, and then dried over anhydrous sodium sulfate. The solvent was distilled off under reduced pressure. The resultant crystals were washed with isopropyl ether, and then recrystallized from a mixed solvent of isopropyl ether and acetonitrile to afford the title compound (23.3 g).
¹H-NMR(CDCl₃)δ:
2.32(3H,s),4.35(2H,q),4.94(1H,d),5.07(1H,d),5.31(2H,bs),6.58(1H,d),7.27-7.42(2H,m),7.54(1H,d),7.74(1H,d),8.14(1H,d)
IR(KBr,cm⁻¹): 3323,1587,1267,1155,1043
MS(FAB,MH⁺): 385
Melting point: 167-168°C

In addition, optical isomers of the title compounds were obtained by the following procedure:
1-Amino-2-[[4-(2,2,2-trifluoroethoxy)-3-methylpyridin-2-yl]methylthio]benzimidazole (8.4 g) was dissolved in ethyl acetate (80 mL), followed by the addition of (+)-diethyl tartrate (4.7 g), tetraisopropyl titanate (3.24 g) and water (41 µL). The resulting mixture was stirred at 50°C for 1.5 hours. The temperature of the thus-obtained mixture was allowed to cool at room temperature,
   N,N-diisopropylethylamine (2.0 mL) and 80% cumene hydroxyperoxide (4.34 g) were added, and the resulting mixture was stirred at room temperature for 1 hour. A saturated aqueous solution of sodium hydrogencarbonate was added to the reaction mixture, followed by stirring for 10 minutes. The thus-obtained mixture was filtered through celite, and the filtrate was extracted with methylene chloride. The organic layer was successively washed with water and brine, and then dried over anhydrous sodium sulfate. The solvent was distilled off under reduced pressure. The residue was purified by chromatography on a silica gel column (silica gel chloroform:methanol = 20:1) to afford (+)-1-amino-2-[[4-(2,2,2-trifluoroethoxy)-3-methylpyridin-2-yl]methylsulfinyl]benzimidazole (8.43 g). On the other hand,
   (-)-1-amino-2-[[4-(2,2,2-trifluoroethoxy)-3-methylpyridin-2-yl]methylsulfinyl]benzimidazole was obtained by repeating the above-described procedure except that (-)-diethyl tartrate was used in lieu of (+)-diethyl tartrate.
   (+)-1-Amino-2-[[4-(2,2,2-trifluoroethoxy)-3-methylpyridin-2-yl]methylsulfinyl]benzimidazole: [α]_{D} = +103 (c=0.51,CHCl₃); MS(El,M⁺): 384; Melting point: 111-113°C
(-)-1-Amino-2-[[4-(2,2,2-trifluoroethoxy)-3-methylpyridin-2-yl]methylsulfinyl]benzimidazole: [α]_{D} = -101 (c=0.51, CHCl₃); MS(El,M⁺): 384; Melting point: 111-113°C

### Example 2

### Synthesis of 1-[N-(2-methylpropyl)amino]-2-[[4-(2,2,2-trifluoroethoxy)-3-methylpyridin-2-yl]methylsulfinyl]benzimidazole

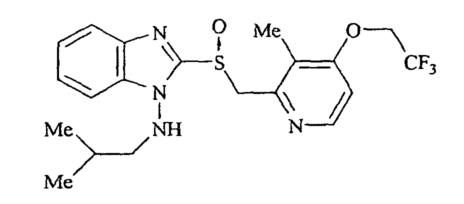

1-[N-(2-Methylpropyl)amino]-2-[[4-(2,2,2-trifluoroethoxy)-3-methylpyridin-2-yl]methylthio]benzimidazole (400 mg) was dissolved in methylene chloride (5 mL), followed by the dropwise addition at -20°C of a solution of 80% m-chloroperbenzoic acid (163 mg) in methylene chloride (5 mL). The resulting mixture was stirred at the same temperature for 1 hour. A saturated aqueous solution of sodium bicarbonate was added to the reaction mixture, the temperature of the thus-prepared mixture was allowed to rise to room temperature, and then, extraction was conducted with chloroform. The organic layer was successively washed with water and brine, and then dried over anhydrous sodium sulfate. The solvent was distilled off under reduced pressure. The residue was purified by chromatography on a silica gel column (silica gel: "NH-DM1020" (product of FUJI SILYSIA CHEMICAL LTD.), hexane:ethyl acetate = 1:1). Recrystallization was then conducted from a mixed solvent of hexane and ethyl acetate to afford the title compound (304 mg).
¹H-NMR(CDCl₃)δ:
1.06(6H,dd),1.87-1.92(1H,m),2.32(3H,s),3.11-3.17(2H,m),4.36(2H,q),4.95(1H,d),5.09(1H,d),5.59(1H,t),6.61(1H,d),7.28-7.41(3H,m),7.78-7.81(1H,m),8.23(1H,d)
Melting point: 129-131°C

Procedures were conducted in a similar manner as in Examples 1 and 2 to obtain the following compounds.

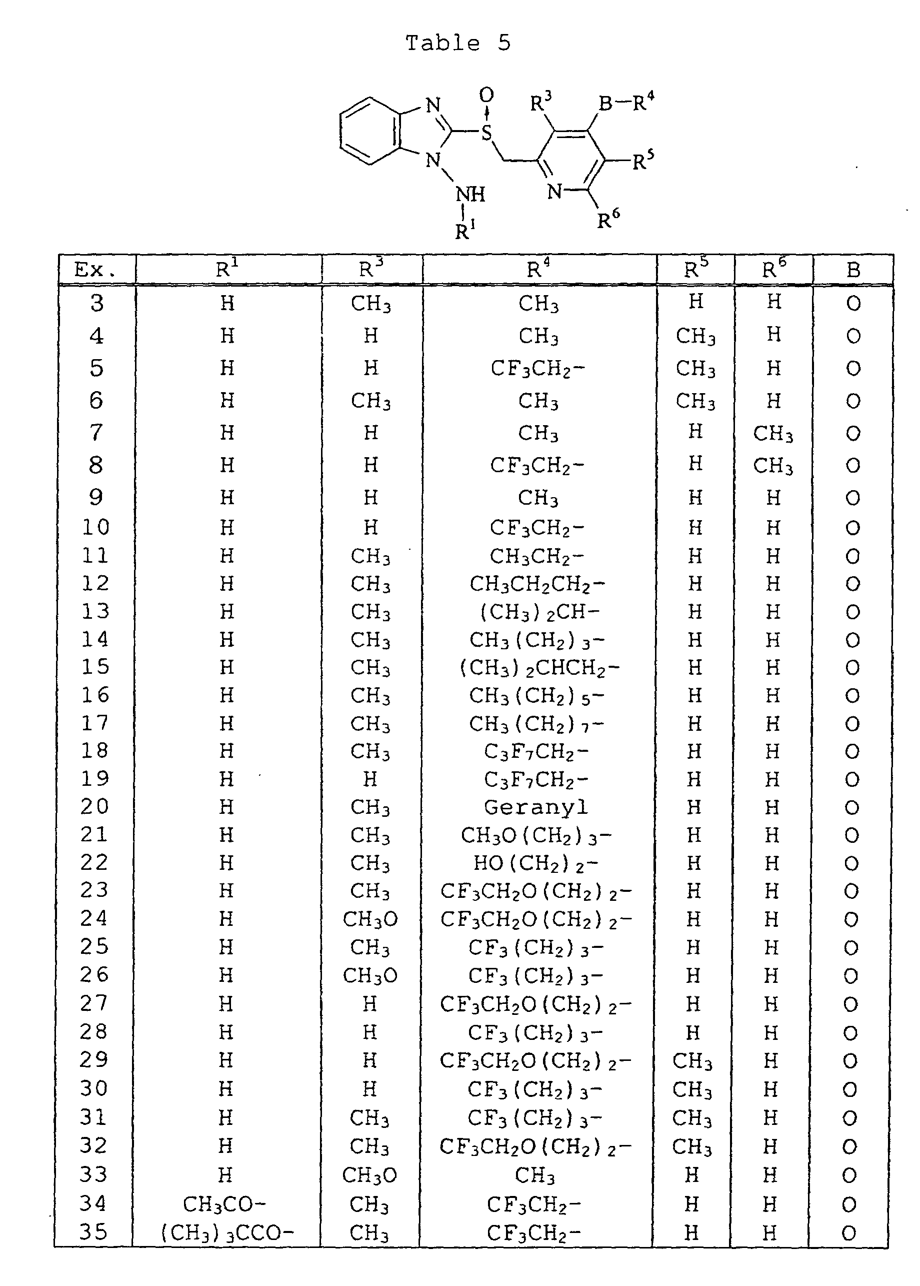

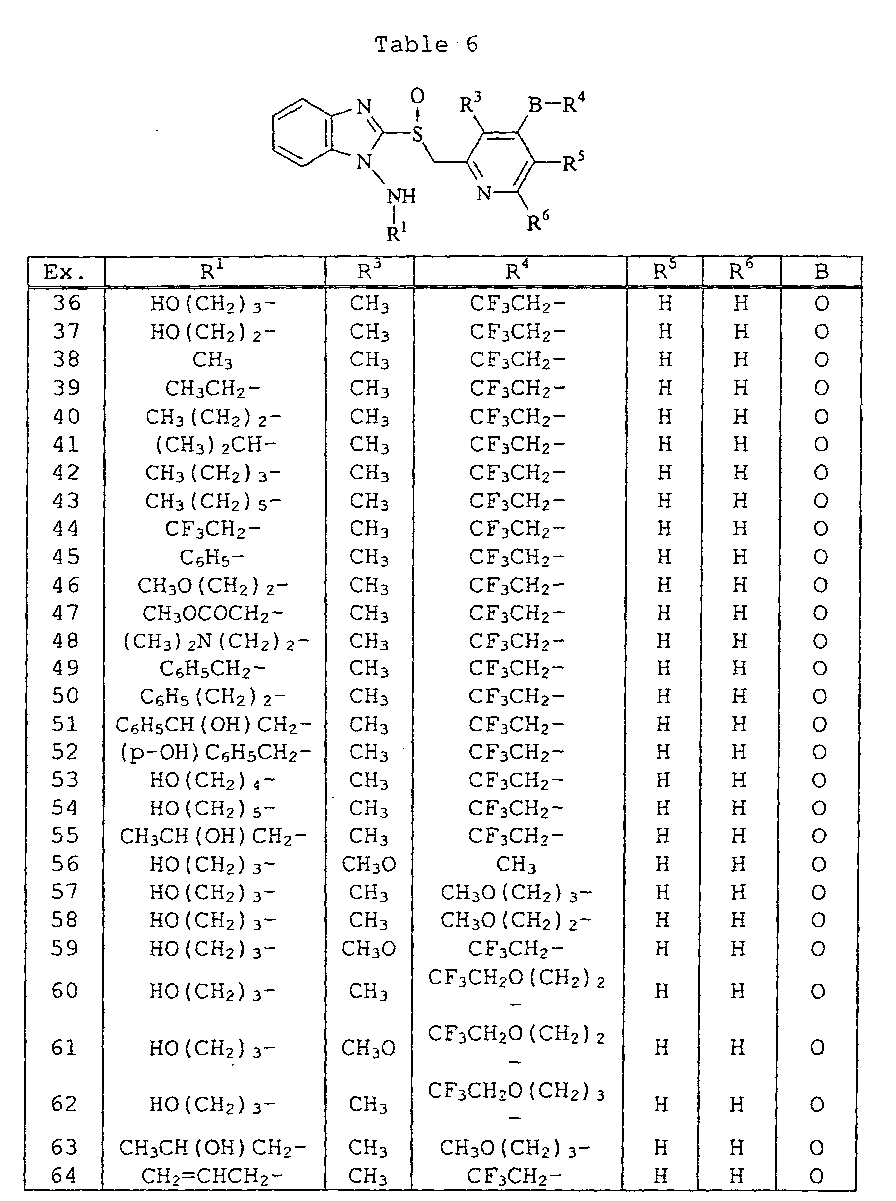

### Example 3

¹H-NMR(CDCl₃)δ:
2.34(3H,s),3.82(3H,s),4.89(1H,d),5.06(1H,d),5.38(2H,s),6.62(1H,d),7.25-7.41(2H,m),7.52-7.56(1H,m),7.71-7.75(1H,m),8.09(1H,d); IR(KBr,cm⁻¹): 3200,1578,1435,1300,1100,1049; MS(FAB,MH⁺): 317; Melting point: 172-173°C

### Example 4

¹H-NMR(CDCl₃)δ:
2.09(3H,s),3.70(3H,s),4.75(1H,d),4.85(1H,d),5.10(2H,s),6.69(1H,s),7.29-7.52(3H,m),7.75-7.78(1H,m),8.07(1H,s); IR(KBr,cm⁻¹): 3157,1597,1458,1296,1149,1039; MS(FAB,MH⁺): 317; Melting point: 146-148°C

### Example 5

¹H-NMR(CDCl₃)δ:
2.25(3H,s),4.16(2H,q),4.77(1H,d),4.82(1H,d),5.08(2H,s),6.64(1H,s),7.30-7.51(3H,m),7.75-7.78(1H,m),8.15(1H,s);IR(KBr,cm⁻¹): 3169,1599,1388,1170,1068,1039; MS (FAB,MH⁺): 385; Melting point: 166-168°C

### Example 6

¹H-NMR(CDCl₃)δ:
2.17(3H,s),2.29(3H,s),3.69(3H,s),4.87(1H,d),5.03(1H,d),5.33(2H,s),7.27-7.41(2H,m),7.52-7.56(1H,m),7.72-7.76(1H,m),8.02(1H,s); IR(KBr,cm⁻¹): 3289,1633,1560,1452,1076,1053; MS(FAB,MH⁺): 331; Melting point: 155-156°C

### Example 7

¹H-NMR(CDCl₃)δ:
2.19(3H,s),3.73(3H,s),4.76(2H,s),5.18(2H,s),6.51(1H,d),6.66(1H,d),7.27-7.54(3H,m),7.72-7.76(1H,m); IR(KBr,cm⁻¹): 3198,1599,1466,1334,1157,1061; MS(FAB,MH⁺): 317; Melting point: 130-133°C

### Example 8

¹H-NMR(CDCl₃)δ:
2.19(3H,s),4.24(2H,q),4.73(1H,d),4.79(1H,d),5.17(2H,s),6.56(1H,d),6.68(1H,d),7.28-7.52(3H,m),7.71-7.75(1H,m); IR(KBr,cm⁻¹): 3198,1597,1458,1269,1165,1049; MS(FAB,MH⁺): 385; Melting point: 163-164°C

### Example 9

¹H-NMR(CDCl₃)δ:
3.74(3H,s),4.77(1H,d),4.88(1H,d),5.12(2H,s),6.71(1H,dd),6.82(1H,d),7.29-7.53(3H,m),7.75-7.79(1H,m),8.25(1H,d); IR(KBr,cm⁻¹): 3159,1595,1298,1045,1028; MS (FAB,MH⁺): 303; Melting point: 141-143°C

### Example 10

¹H-NMR(CDCl₃)δ:
4.25(2H,dq),4.80(1H,d),4.86(1H,d),5.14(2H,s),6.76(1H,dd),6.86(1H,d),7.29-7.52(3H,m),7.74-7.77(1H,m),8.29(1H,d); IR(KBr,cm⁻¹): 3169,1595,1452,1271,1178,1070,1037; MS(FAB,MH⁺): 371; Melting point: 126-127°C

### Example 11

¹H-NMR(CDCl₃)δ:
1.42(3H,t),2.23(3H,s),4.02(2H,q),4.89(1H,d),5.05(1H,d),5.40(2H,s),6.58(1H,d),7.25-7.75(4H,m),8.06(1H,d); IR(KBr,cm⁻¹): 1579,1464,1300,1074,1024; MS(FAB,MH⁺): 331; Melting point: 160-161°C

### Example 12

¹H-NMR(CDCl₃)δ:
1.03(3H,t),1.76-1.88(2H,m),2.25(3H,s),3.91(2H,t),4.90(1H,d),5.07(1H,d),5.38(2H,s),6.59(1H,d),7.26-7.76(4H,m),8.07(1H,d); IR(KBr,cm⁻¹): 1581,1464,1298,1082,1053; MS(FAB,MH⁺):345; Melting point: 137-138°C

### Example 13

¹H-NMR(CDCl₃)δ:
1.32(6H,dd),2.20(3H,s),4.50-4.59(1H,m),4.88(1H,d),5.05(1H,d),5.40(2H,s),6.58(1H,d),7.25-7.-75(4H,m),8.05(1H,d); IR(KBr,cm⁻¹): 1578,1464,1294,1082,1049; MS(FAB,MH⁺): 345; Melting point: 155-156°C

### Example 14

¹H-NMR(CDCl₃)δ:
0.97(3H,t),1.41-1.83(4H,m),2.24(3H,s),3.95(2H,t),4.89(1H,d),5.07(1H,d),5.38(2H,s),6.60(1H,d),7.29-7.41(2H,m),7.53-7.56(1H,m),7.73-7.76(1H,m),8.06(1H,d); IR(KBr,cm⁻¹): 1581,1458,1298,1088,1037; MS(FAB,MH⁺): 359; Melting point: 123-125°C

### Example 15

¹H-NMR(CDCl₃)δ:
1.02(6H,d),2.05-2.15(2H,m),2.25(3H,s),3.70(2H,d),4.89(1H,d),5.05(1H,d),5.41(2H,s),6.58(1H,d),7.25-7.75(4H,m),8.06(1H,d); IR(KBr,cm⁻¹): 1579,1458,1298,1086,1026; MS(FAB,MH⁺): 359; Melting point: 154-156°C

### Example 16

¹H-NMR(CDCl₃)δ:
0.91(3H,t)1.30-1.50(6H,m)1.76-1.84(2H,m)2.24(3H,s)3.94(2H,t),4.89(1H,d),5.06(1H,d),5.39(2H,s),6.59(1H,d),7.26-7.76(4H,m),8.06(1H,d); IR(KBr,cm⁻¹): 1581,1458,1298,1088,1037; MS(FAB,MH⁺): 387; Melting point: 131-132°C

### Example 17

¹H-NMR(CDCl₃)δ:
0.89(3H,t),1.29-1.47(10H,m),1.73-1.84(2H,m),2.24(3H,s),3.94(2H,t),4.89(1H,d),5.07(1H,d),5.38(2H,s), 6.59(1H,d),7.26-7.76(4H,m),8.06(1H,d); IR(KBr,cm⁻¹):
1581,1468,1300,1088,1037; MS(FAB,MH⁺): 415; Melting point: 118-119°C

### Example 18

¹H-NMR(DMSO-d₆)δ:
2.23(3H,s),4.88(2H,t),5.00(2H,t),6.39(2H,s),7.13(1H,d),7.32(1H,t),7.41(1H,t),7.61(1H,d),7.73(1H,d),8.30(1H,d); IR (KBr, cm⁻¹): 3325,1581,1126,1007; MS (FAB,MH⁺): 425; Melting point: 195-198°C (decomposed)

### Example 19

¹H-NMR(CDCl₃)δ:
4.33-4.43(2H,m)4.81(1H,d),4.87(1H,d),5.14(2H, s),6.77(1H,dd),6.90(1H,d),7.29-7.78(4H,m),8.29(1H,d); IR(KBr,cm⁻¹): 1595,1317,1229,1127,1055,1022; MS(FAB,MH⁺): 471; Melting point: 72-74°C

### Example 20

¹H-NMR(CDCl₃)δ:
1.60(3H,s),1.66(3H,s),1.71(3H,s),2.05-2.17(4H,m),2.24(3H,s),4.54(2H,d),4.89(1H,d),5.07(1H,d),5.06-5.09(1H,m),5.38(2H,s),5.40-5.42(1H,m),6.60(1H,d),7.26-7.76(4H,m),8.06(1H,d); IR (KBr, cm⁻¹):1578,1458,1292,1080,1034;MS(FAB,MH⁺): 439; Melting point: 99-101°C

### Example 21

¹H-NMR(CDCl₃)δ:
2.05(2H,t),2.24(3H,s),3.34(3H,s),3.53(2H,t),4.05(2H,t),4.90(1H,d),5.06(1H,d),5.37(2H,s),6.62(1H,d),7.27-7.56(3H,m,7.72-7.76(1H,m),8.07(1H,d); IR(KBr,cm⁻¹): 3316,1581,1458,1296,1116,1037; MS(FAB,MH⁺): 375; Melting point: 131-133°C

### Example 22

¹H-NMR(DMSO-d₆)δ:
2.24(3H,s),3.73-3.77(2H,m),4.07(2H,t),4.82(1H,d),4.90(1H,t),4.94(1H,d),6.37(2H,s),6.92(1H,d),7.30-7.40(2H,m),7.59-7.74(2H,m),8.19(1H,d); MS(FAB,MH⁺): 347; Melting point: 159-160°C (decomposed)

### Example 23

¹H-NMR (CDCl₃)δ:
2.27(3H,s),3.94(2H,q),3.98-4.02(2H,m),4.12-4.18(2H,m),4.92(1H,d),5.08(1H,d),5.36(2H,s),6.60(1H,d),7.27-7.54(3H,m),7.73-7.79(1H,m),8.09(1H,d); MS(EI,M⁺): 428; Melting point: 120-121°C

### Example 24

¹H-NMR(CDCl₃)δ:
3.88-4.02(4H,m),3.94(3H,s),4.17-4.20(2H,m),4.95(1H,d),5.02(1H,d),5.37(2H,s),6.71(1H,d),7.29-7.56(3H,m),7.71-7.75(1H,m)8.00(1H,d); MS (EI,M⁺): 444

### Example 25

¹H-NMR(CDCl₃)δ:
2.04-2.35(4H,m),2.26(3H,s),4.02(2H,t),4.91(1H,d),5.05(1H,d),5.36(2H,s),6.58(1H,d),7.29-7.56(3H,m),7.72-7.75(1H,m),8.09(1H,d); MS(EI,M⁺): 412; Melting point: 146-147°C

### Example 26

¹H-NMR (CDCl₃)δ:
2.05-2.38(4H,m),3.93(3H,s),4.07(2H,t),4.97(1H,d),5.03(1H,d),5.36(2H,s),6.69(1H,d),7.25-7.57(3H,m),7.72-7.75(1H,m),8.01(1H,d); MS(EI,M⁺): 428; Melting point: 98-100°C

### Example 27

¹H-NMR(CDCl₃)δ:
3.89(2H,q),3.88-3.96(2H,m),4.05-4.08(2H,m),4.77(1H,d),4.85(1H,d),5.15(2H,s),6.72(1H,dd),6.84(1H,d),7.26-7.52(3H,m),7.74-7.77(1H,m),8.23(1H,d); MS(EI,M⁺): 414; Melting point: 108-110°C

### Example 28

¹H-NMR(CDCl₃)δ:
1.96-2.31(4H,m),3.88-3.94(2H,m),4.77(1H,d),4.85(1H,d),5.10(2H,s),6.69(1H,dd),6.78(1H,d),7.26-7.53(3H,m),7.75-7.79(1H,m),8.25(1H,d); MS(EI,M⁺): 398; Melting point: 141-143°C

### Example 29

¹H-NMR(CDCl₃)δ:
2.11(3H,s),3.90(2H,q),3.93-4.00(4H,m),4.74(1H,d),4.82(1H,d),5.09(2H,s),6.66(1H,s),7.26-7.50(3H,m),7.75-7.78(1H,m),8.09(1H,s); MS(EI,M⁺): 428; Melting point: 137-139°C

### Example 30

¹H-NMR(CDCl₃)δ:
1.95-2.28(4H,m),2.10(3H,s),3.79-3.87(2H,m),4.75(1H,d),4.82(1H,d),5.06(2H,s),6.62(1H,s),7.30-7.49(3H,m),7.74-7.76(1H,m),8.10(1H,s); MS(EI,M⁺): 412; Melting point: 154-155°C

### Example 31

¹H-NMR(CDCl₃)δ:
2.00-2.40(4H,m),2.14(3H,s),2.26(3H,s),3.75(2H,t),4.88(1H,d),5.00(1H,d),5.34(2H,s),7.27-7.53(3H,m),7.70-7.72(1H,m),8.03(1H,s); MS(EI,M⁺): 426; Melting point: 160-161°C

### Example 32

¹H-NMR(CDCl₃)δ:
2.17(3H,s),2.29(3H,s),3.88-3.98(6H,m),4.87(1H,d),5.02(1H,d),5.33(2H,s),7.29-7.55(3H,m),7.71-7.75(1H,m),8.03(1H,s); MS(EI,M⁺):442; Melting point: 123-124°C

### Example 33

¹H-NMR(CDCl₃)δ:
3.88(3H,s),3.93(3H,s),4.96(1H,d),5.02(1H,d),5.38(2H,s),6.74(1H,d),7.26-7.42(2H,m),7.54-7.57(1H,m),7.73-7.76(1H,m),8.03(1H,d); IR(KBr,cm⁻¹):
3250,1589,1493,1309,1068,1028,1001; MS(FAB,MH⁺): 333; Melting point: 188-189°C

### Example 34

¹H-NMR(DMSO-d₆)δ:
1.99(3H,s),2.17(3H,s),4.67(1H,d),4.91(2H,q),5.05(1H,d),7.10(1H,d),7.36-7.52(3H,m),7.83(1H,q),8.32(1H,d);
IR (KBr, cm⁻¹): 3184,1678,1581,1265,1169, 1034; MS (FAB,MH⁺): 427; Melting point: 176-177°C (decomposed)

### Example 35

¹H-NMR(CDCl₃)δ:
1.37(9H,s),2.24(3H,s),4.36(2H,q),4.82(1H,d),4.97(1H,d),6.63(1H,d),7.26-7.37(3H,m),7.67(1H,d),8.29(1H,d),9.72(1H,s); IR(KBr,cm⁻¹): 3192,1686,1579,1477,1263,1167,1049;
MS(FAB,MH⁺): 469; Melting point: 117-118°C (decomposed)

### Example 36

¹H-NMR(CDCl₃)δ:
1.78-1.94(2H,m),2.37(3H,s),3.40-3.58(2H,m),3.78-3.98(3H,m),4.39(2H,q),4.95(1H,d),5.02(1H,d),5.62(1H,t),6.65(1H,d),7.24-7.55(3H,m),7.80-7.83(1H,m),8.27(1H,d); IR(KBr,cm⁻¹): 3339,3249,2940,2874,1588,1482,1458,1431,1385,1318,1271,1163,1111,1061,1005,976,820,766,750; MS(FAB,MH⁺):443;
Melting point: 122-125°C
Optical isomers: [α]_{D} of the (+) isomer = +101(c=0.53, CHCl₃): [α]_{D} of the (-) isomer: = -92(c=0.53,CHCl₃)

### Example 37

¹H-NMR(CDCl₃)δ:
2.37(3H,s),3.48-3.71(4H,m),4.14(1H,t),4.37(2H,d),5.04(1H,d),5.20(1H,d),5.72(1H,t),6.59(1H,d),7.31-7.46(2H,m),7.55(1H,d),7.79(1H,d),8.17(1H,d); IR(KBr,cm⁻¹):
3320,1587,1477,1265,1169,1111,1053,980; MS(FAB,MH⁺): 429;
Melting point: 133.5-137.5°C

### Example 38

¹H-NMR(CDCl₃)δ:
2.33(3H,s),3.10(3H,d),4.37(2H,q),4.95(1H,d),5.02(1H,d),5.60(1H,q),6.62(1H,d),7.30-7.54(3H,m),7.79-7.82(1H,m),8.24(1H,d); IR(KBr,cm⁻¹): 3200,1581,1460,1269,1174,1032;
MS(FAB,MH⁺): 399; Melting point: 147-148°C

### Example 39

¹H-NMR (CDCl₃)δ:
1.22(3H,t),2.33(3H,s),3.38(2H,q),4.37(2H,q),4.95(1H,d),5.09(1H,d),6.61(1H,d),7.24-7.81(5H,m),8.22(1H,d);
IR(KBr,cm⁻¹): 3227,1578,1001; MS(FAB,MH⁺): 413; Melting point: 100-101°C

### Example 40

¹H-NMR(CDCl₃)δ:
1.02(3H,t),1.57-1.70(2H,m),2.32(3H,s),3.30(2H,q),4.37(2H,q),4.95(1H,d),5.09(1H,d),5.62(1H,t),6.61(1H,d),7.26-7.54(3H,m),7.78-7.81(1H,m),8.22(1H,d); IR(KBr,cm⁻¹):
3219,1579,1458,1259,1174,1113,1007,980; MS(FAB,MH⁺): 426;
Melting point: 96-98°C

### Example 41

¹H-NMR(CDCl₃)δ:
1.16(6H,dd),2.32(3H,s),3.75-3.80(1H,m),4.36(2H,q),4.93(1H,d),5.13(1H,d),5.61(1H,d),6.60(1H,d),7.27-7.54(3H,m),7.77-7.80(1H,m),8.21(1H,d); IR(KBr,cm⁻¹):
3220,1581,1477,1325,1147,1030; MS(FAB,MH⁺): 427; Melting point: 158-159°C

### Example 42

¹H-NMR(CDCl₃)δ:
0.94(3H,t),1.42-1.64(4H,m),2.32(3H,s),3.33(2H,q),4.36(2H,q),4.95(1H,d),5.09(1H,d),5.94(1H,t),6.61(1H,d),7.26-7.53(3H,m),7.78-7.81(1H,m),8.23(1H,d); IR(KBr,cm⁻¹):
3240,1578,1458,1300,1257,1172,1109,1003; MS (FAB,MH⁺): 441; Melting point: 102-103°C

### Example 43

¹H-NMR(CDCl₃)δ:
0.89(3H,t),1.30-1.61(8H,m),2.32(3H,s),3.32(2H,q),4.36(2H,q),4.94(1H,d),5.09(1H,d),5.60(1H,t),6.61(1H,d),7.29-7.53(3H,m),7.78-7.81(1H,m),8.23(1H,d); IR(KBr,cm⁻¹): 3283,1581,1458,1263,1170,1045; MS(FAB,MH⁺): 469; Melting point: 90-91°C

### Example 44

¹H-NMR(CDCl₃)δ:
2.30(3H,s),3.86-3.93(2H,m),4.36(2H,q),4.87(1H,d),5.17(1H,d),6.42(1H,bt),6.62(1H,d),7.29-7.77(4H,m),8.18(1H,d);
IR(film, cm⁻¹): 3223,1582,1051; MS (EI,M⁺): 466

### Example 45

¹H-NMR(DMSO-d₆)δ:
2.22(3H,s),4.87(2H,q),5.00(2H,bs),6.58-8.19(11H,m),9.81(1H,bs); IR(KBr,cm⁻¹): 3193,1603,1579,1059; MS(EI,M⁺): 460;
Melting point: 105-107°C

### Example 46

¹H-NMR(DMSO-d₆)δ:
2.26(3H,s),3.23(3H,s),3.31-3.35(2H,m),3.44-3.48(2H,m),4.88(1H,d),4.89(2H,q),5.02(1H,d),7.06(1H,dd),7.16(1H,t),7.28-7.43(2H,m),7.66(1H,d),7.75(1H,d),8.24(1H,d);MS(FAB,MH⁺): 443; Melting point: 146-147°C

### Example 47

¹H-NMR(CDCl₃)δ:
2.30(3H,s),3.80(3H,s),4.12(2H,d),4.37(2H,q),4.95(1H,d),5.11(1H,d),6.16(1H,t),6.61(1H,d),7.32-7.58(3H,m),7.78-7.80(1H,m),8.22(1H,d); IR(KBr,cm⁻¹): 3211,1753,1578,1477,1261,1165,1107,1012; MS(EI,M⁺): 456;
Melting point: 143-144°C

### Example 48

¹H-NMR(CDCl₃)δ:
2.33(9H, s),2.57-2.61(2H,m),3.34-3.40(2H,m),4.36(2H,q),4.93(1H,d),5.06(1H,d),5.91(1H,t),6.60(1H,d),7.26-7.55(3H,m),7.80-7.83(1H,m),8.24(1H,d); MS(EI,M⁺): 455; Melting point: 146-148°C

### Example 49

¹H-NMR(CDCl₃)δ:
2.31(3H,s),4.35(2H,q),4.42(2H,d),4.92(1H,d),5.00(1H,d),5.89(1H,t),6.60(1H,d),7.28-7.46(8H,m),7.77-7.81(1H,m),8.21(1H,d); IR(KBr,cm⁻¹): 3216,1578,1460,1258,1165,1111,1001;
MS(EI,M⁺): 474; Melting point: 150-151°C (decomposed)

### Example 50

¹H-NMR(CDCl₃)δ:
2.31(3H,s),2.96(2H,t),3.60(2H,q),4.35(2H,q),4.92(1H,d),5.07(1H,d),5.58(1H,t),6.57(1H,d),7.27-7.32(8H,m),7.76-7.80(1H,m),8.15(1H,d); IR(KBr,cm⁻¹):
3210, 1578,1458, 1260,1174,1113,1009; MS(EI,M⁺): 488; Melting point: 154-155°C (decomposed)

### Example 51

¹H-NMR(CDCl₃)δ:
2.36(1.5H,s),2.38(1.5H,s),3.43-3.52(1H,m),3.60-3.67(1H,m),4.34(1H,q),4.37(1H,q),4.51(1H,s),4.82-4.93(1H,m),5.02(0.5H,d),5.04(0.5H,d),5.15(0.5H,d),5.27(0.5H,d),5.74(0.5H,t),5.90(0.5H,t),6.53(0.5H,d),6.61(0.5H,d),7.25-7.43(7H,m),7.50-7.55(1H,m),7.76-7.83(1H,m),8.03(0.5H,d),8.20(0.5H,d);
MS (EI,M⁺): 504

### Example 52

¹H-NMR(DMSO-d₆)δ:
2.23(3H,s),4.17(2H,d),4.69(1H,d),4.89(2H,q),4.96(1H,d),6.67(2H,d),7.05-7.38(6H,m),7.66(1H,d),7.75(1H,d),8.27(1H,d),9.34(1H,s); MS(FAB,MH⁺): 491; Melting point: 105-111°C (decomposed)

### Example 53

¹H-NMR(DMSO-d₆)δ:
1.45-1.55(4H,m),2.25(3H,s),3.12-3.45(4H,m),4.40(1H,t),4.88(2H,q),4.92(1H,d),4.99(1H,d),7.06(1H,d),7.07(1H,t),7.29-7.43(2H,m),7.67-7.78(2H,m),8.23(1H,d); MS(EI,M⁺): 456;
Melting point: 136-137°C

### Example 54

¹H-NMR(DMSO-d₆)δ:
1.35-1.50(6H,m),2.25(3H,s),3.12-3.38(4H,m),4.33(1H,t),4.89(2H,q),4.92(1H,d),4.99(1H,d),7.05(1H,d),7.06(1H,t),7.29-7.43(2H,m),7.66-7.78(2H,m),8.22(1H,d); MS(EI,M⁺): 470;
Melting point: 159-160°C

### Example 55

¹H-NMR(CDCl₃)δ:
1.17(1.5H,d),1.19(1.5H,d),2.34(1.5H,s),2.36(1.5H,s),3.10-3.25(1H,m),3.45-3.60(1H,m),3.85-4.10(2H,m),4.35(1H,q),4.38(1H,q),4.95-5.28(2H,m),5.66-5.75(0.5H,m),5.82-5.92(0.5H,m),6.55(0.5H,d),6.63(0.5H,d),7.22-7.48(3H,m),7.50-7.60(1H,m),7.75(0.5H,d),7.80(0.5H,d); MS(FAB,MH⁺): 443

### Example 56

¹H-NMR(CDCl₃)δ:
1.80-1.86(2H,m),3.37-3.49(2H,m),3.78-3.85(2H,m),3.92(6H,s),4.45(1H,t),4.90(1H,d),5.02(1H,d),6.31(1H,t),6.84(1H,d),7.29-7.41(2H,m),7.63-7.81(2H,m),8.14(1H,d); MS(EI,M⁺): 390; Melting point: 168-169°C

### Example 57

¹H-NMR(CDCl₃)δ:
1.83-1.88(2H,m),2.05-2.12(2H,m),2.31(3H,s),3.36(3H,s),3.46-3.58(4H,m),3.91-3.95(2H,m),4.07-4.20(3H,m),4.89(1H,d),4.98(1H,d),5.65(1H,t),6.70(1H,d),7.31-7.56(3H,m),7.81-7.84(1H,m),8.20(1H,d); MS(FAB,MH⁺): 433
Optical isomers: [α]_{D} of the (+) isomer = +111(c=0.39, CHCl₃); [α]_{D} of the (-) isomer = -127(c=0.38,CHCl₃)

### Example 58

¹H-NMR (CDCl₃)δ:
1.83-1.87(2H,m),2.34(3H,s),3.43-3.52(2H,m),3.45(3H,s),3.76-3.80(2H,m),3.90-3.94(2H,m),4.13-4.16(3H,m),4.92(1H,d),4.98(1H,d),5.65(1H,t),6.68(1H,d),7.24-7.55(3H,m),7.81-7.84(1H,m),8.20(1H,d); MS(EI,M⁺): 418; Melting point: 97-99°C

### Example 59

¹H-NMR(CDCl₃)δ:
1.80-1.90(2H,m),3.40-3.60(2H,m),3.65(1H,t),3.89-3.93(2H,m),3.99(3H,s),4.43(2H,q),4.95(1H,d),5.09(1H,d),5.57(1H,t),6.77(1H,d),7.34-7.57(3H,m),7.81-7.84(1H,m),8.17(1H,d);
MS(EI,M⁺): 458

### Example 60

¹H-NMR(CDCl₃)δ:
1.84-1.88(2H,m),2.33(3H,s),3.47-3.53(2H,m),3.91-4.04(7H,m),4.16-4.20(2H,m),4.92(1H,d),5.00(1H,d),5.62(1H,t),6.68(1H,d),7.30-7.56(3H,m),7.81-7.84(1H,m),8.22(1H,d);
MS(EI,M⁺): 486

### Example 61

¹H-NMR(CDCl₃)δ:
1.80-1.90(2H,m),3.47-3.50(2H,m),3.90-4.05(7H,m),3.93(3H,s),4.21-4.24(2H,m),4.92(1H,d),5.07(1H,d),5.64(1H,t),6.78(1H,d),7.33-7.56(3H,m),7.80-7.83(1H,m),8.12(1H,d);
MS (EI,M⁺): 502

### Example 62

¹H-NMR(CDCl₃)δ:
1.82-1.90(2H,m),2.04-2.17(2H,m),2.31(3H,s),3.47-3.54(2H,m),3.78-3.93(6H,m),4.09-4.14(3H,m),4.90(1H,d),4.99(1H,d),5.63(1H,t),6.70(1H,d),7.27-7.56(3H,m),7.81-7.84(1H,m),8.21 (1H,d); MS(EI,M⁺): 500

### Example 63

¹H-NMR(CDCl₃)δ:
1.17(1.5H,d),1.18(1.5H,d),2.03-2.12(2H,m),2.28(1.5H,s),2.30(1.5H,s),3.11-3.20(1H,m),3.32(1.5H,s),3.33(1.5H,s),3.45-3.67(3H,m),3.85-4.20(4H,m),4.94-5.24(2H,m),5.76-5.80(0.5H,m),5.95-6.02(0.5H,m),6.59(0.5H,d),6.67(0.5H,d),7.26-7.55(3H,m),7.72-7.80(1H,m),8.00(0.5H,d),8.17(0.5H,d);
MS (EI,M⁺): 432

### Example 64

¹H-NMR(CDCl₃)δ:
2.32(3H,s),3.94(2H,ddt),4.37(2H,q),4.96(1H,d),5.07(1H,d),5.18(1H,ddd),5.22(1H,ddd),5.72(1H,t),6.02(1H,ddt),6.62(1H,d),7.26-7.81(4H,m),8.24(1H,d); IR(KBr,cm⁻¹): 3218,1588,1578,1460,1175,1111,1010; MS(EI,M⁺): 424,320,106; Melting point: 126-127° (decomposed)

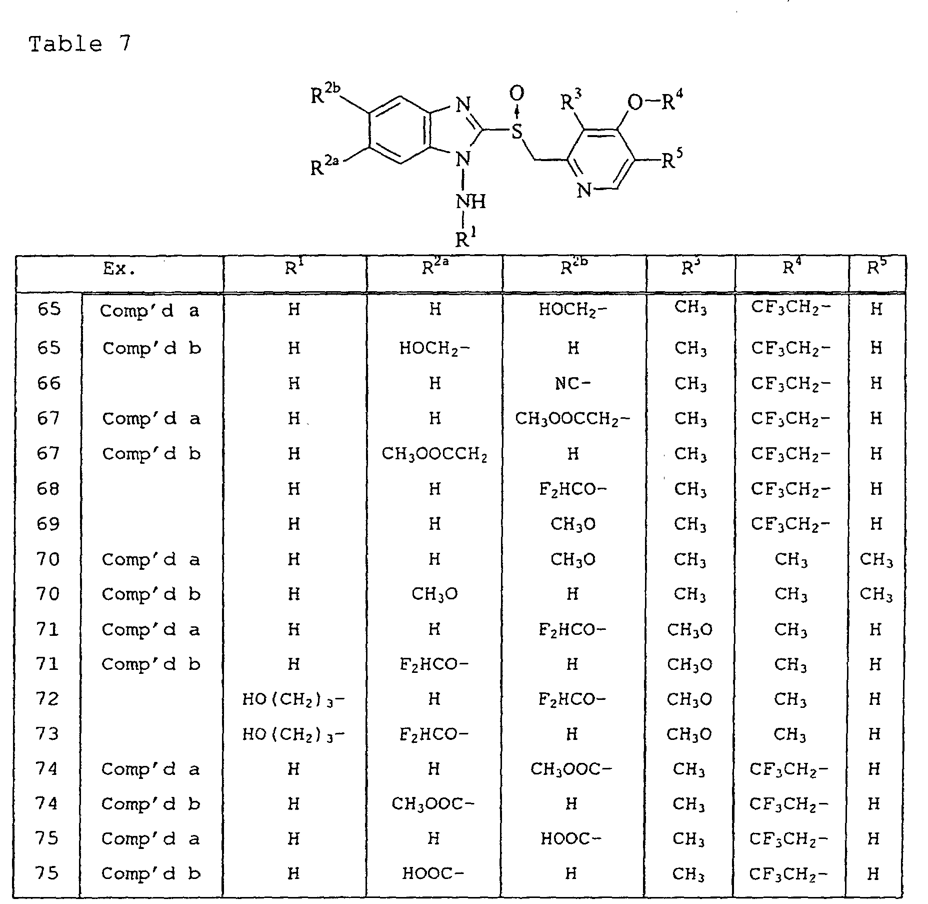

### Example 65 (1:1 Mixture of Compound a and Compound b)

¹H-NMR(DMSO-d₆)δ:
2.32(3H,s),3.14(0.5H,t),3.39(0.5H,t),4.38(2H,q),4.78(1H,d),4.83(1H,d),4.94(0.5H,d),4.95(0.5H,d),5.05(0.5H,d),5.06(0.5H,d),5.43(1H,s),5.46(1H,s),6.60(1H,d),7.27-7.71(3H,m),8.15(0.5H,d),8.16(0.5H,d); IR(KBr,cm⁻¹): 3300,1581,1263,1163,1109,1010,966; MS(FAB,MH⁺): 415;
Melting point: 160-161°C

### Example 66

¹H-NMR(DMSO-d₆)δ:
2.23(3H,s),4.89(2H,q),4.93(2H,s),6.52(2H,s),7.05(1H,d),7.78(2H,s),8.22(1H,d),8.31(1H,s); IR(KBr,cm⁻¹):
3318,2226, 1581,1043; MS(EI,M⁺): 407; Meltingpoint: 180-181°C (decomposed)

### Example 67 (1:1 Mixture of Compound a and Compound b)

¹H-NMR(DMSO-d₆)δ:
2.32(3H,s),3.69(1.5H,s),3.70(1.5H,s),3.74(1H,s),3.78(1H,s),4.36(2H,q),4.92(1H,d),5.06(0.5H,d),5.07(0.5H,d),5.32(1H,s),5.34(1H,s),6.58(1H,d),7.19-7.69(3H,m),8.15(1H,d);
MS(EI,M⁺): 456; Melting point: 152-153°C

### Example 68

¹H-NMR(DMSO-d₆)δ:
2.24(3H,s),4.86(1H,d),4.88(2H,q),4.96(1H,d),6.43(2H,s),7.07(1H,d),7.23(1H,t),7.26(1H,dd),7.54(1H,d),7.63(1H,d),8.26(1H,d); IR(KBr,cm⁻¹): 1581,1458,1263,1163,1113,1045;
MS(FAB,MH⁺): 451; Melting point: 194-195°C

### Example 69

¹H-NMR(DMSO-d₆)δ:
2.25(3H,s),3.80(3H,s),4.82(1H,d),4.89(2H,q),4.98(1H,d),6.35(2H,bs),7.04(1H,dd),7.06(1H,d),7.23(1H,d),7.49(1H,d),8.29(1H,d); IR(KBr,cm⁻¹): 3310,1619,1581,1009; MS(EI,M⁺): 412; Melting point: 160-162°C (decomposed)

### Example 70 (1:1 Mixture of Compound a and Compound b)

¹H-NMR(DMSO-d₆)δ:
2.19(3H,s),2.28(3H,s),3.71(3H,s),3.80(1.5H,s),3.84(1.5H,s),4.77(1H,d),4.91(1H,d),6.28(1H,s),6.32(1H,s),6.89-7.23(2H,m)7.47-7.62(1H,m),8.16(1H,s); MS(EI,M⁺): 360; Melting point: 160-162°C

### Example 71 (1:1 Mixture of Compound a and Compound b)

¹H-NMR(CDCl₃)δ:
3.88(3H,s),3.93(3H,s),4.92(1H,d),5.02(0.5H,d),5.03(0.5H,d),5.41(1H,s),5.42(1H,s),6.52(0.5H,t),6.57(0.5H,t),6.74(1H,d),7.08(0.5H,dd),7.20(0.5H,dd),7.32(0.5H,d),7.48(0.5H,d),7.53(0.5H,d),7.68(0.5H,d),8.01(0.5H,d),8.02(0.5H,d);
MS(EI,M⁺): 398; Melting point: 137-139°C

### Example 72

¹H-NMR(CDCl₃)δ:
1.80-1.88(2H,m),3.40-3.45(2H,m),3.87-4.00(3H,m),3.89(3H,s),3.91(3H,s),4.88(1H,d),5.03(1H,d),5.88(1H,t),6.53(1H,t),6.79(1H,d),7.17(1H,dd),7.49-7.53(2H,m),8.11(1H,d);
MS(EI,M⁺): 456

### Example 73

¹H-NMR(DMSO-d₆)δ:
1.58-1.63(2H,m),3.15-3.33(3H,m),3.47-3.54(2H,m),3.82(3H,s),3.89(3H,s),4.50(1H,t),4.77(1H,d).4.97(1H,d),7.08(1H,d),7.17(1H,dd),7.30(1H,t),7.50(1H,d),7.83(1H,d),8.13(1H,d);
MS(EI,M⁺): 456

### Example 74 (1:1 Mixture of Compound a and Compound b)

¹H-NMR(CDCl₃)δ:
2.32(3H,s),3.95(3H,s),4.36(2H,q),4.91(1H,d),5.11(1H,d),5.39(2H,s),6.58(1H,d),7.75(1H,d),7.99(1H,dd),8.09(1H,d),8.29(1H,d); IR(KBr,cm⁻¹):
3329,1714,1578,1255,1163,1113,1022,974; MS(FAB,MH⁺): 443;
Melting point: 153.4-153.6°C

### Example 75 (1:1 Mixture of Compound a and Compound b)

¹H-NMR(DMSO-d₆)δ:
2.25(3H,s),4.85-4.99(4H,m),6.48(1H,s),6.50(1H,s),7.06(1H,d),7.68(0.5H,d),7.79(0.5H,d),7.89(0.5H,dd),8.00(0.5H,dd),8.25(1H,d),8.28(1H,d),12.80(1H,bs); IR(KBr,cm⁻¹):
3345,1695,1587,1477,1263,1163,1111,1062,966; MS(FAB,MH⁺): 429; Melting point: 240-250°C (decomposed)

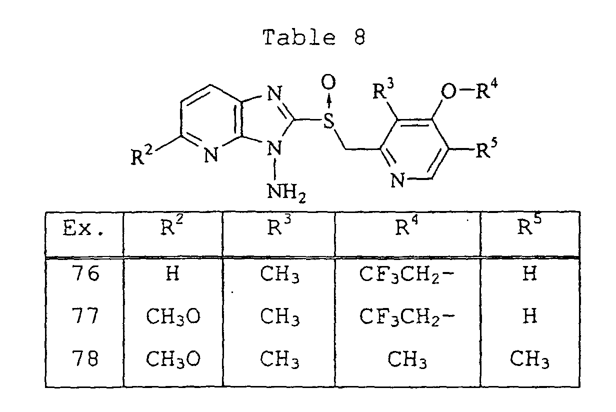

### Example 76

¹H-NMR(DMSO-d₆)δ:
2.24(3H,s),4.90(2H,q),4.91(2H,s),6.34(2H,s),7.06(1H,d),7.40(1H,dd),8.18(1H,dd),8.24(1H,d),8.51(1H,dd); IR(KBr,cm⁻¹): 1579,1481,1298,1267,1163,1109,1049;
MS(FAB,MH⁺): 386; Melting point: 190-191°C (decomposed)

### Example 77

¹H-NMR(CDCl₃)δ:
2.35(3H,s),4.04(3H,s),4.36(2H,q),4.98(1H,d),5.06(1H,d),5.25(2H,s),6.57(1H,d),6.74(1H,d),7.90(1H,d),8.15(1H,d); IR(KBr, cm⁻¹): 1581,1373,1261,1111,1049,1028; MS (FAB,MH⁺): 416; Melting point: 174-175°C (decomposed)

### Example 78

¹H-NMR(CDCl₃)δ:
2.18(3H,s),2.34(3H,s),3.73(3H,s),4.04(3H,s),4.92(1H,d),5.20(1H,d),5.25(2H,s),6.74(1H,d),7.91(1H,d),8.04(1H,s);
MS(FAB,MH⁺): 362; Melting point: 174-175°C

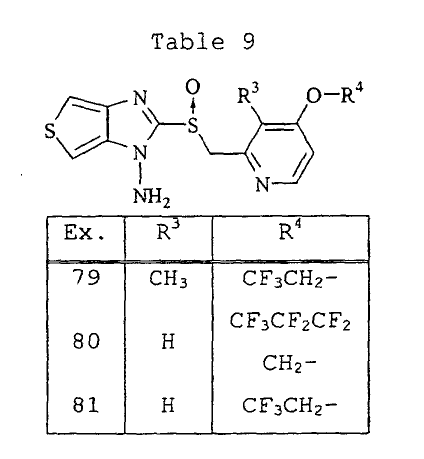

### Example 79

¹H-NMR(DMSO-d₆)δ:
2.23(3H,s),4.85(2H,s),4.91(2H,q),6.16(2H,s),7.01(1H,d),7.08(1H,d),7.43(1H,d),8.28(1H,d); IR(KBr,cm⁻¹): 3320,3156,1581,1467,1267,1113,1043; MS(FAB,MH⁺): 391;
Melting point: 157-160°C (decomposed)

### Example 80

¹H-NMR(DMSO-d₆)δ:
4.68(1H,d),4.76(1H,d),4.97(2H,t),6.16(2H,s),7.02(1H,d),7.10(1H,dd),7.17(1H,d),7.44(1H,d),8.44(1H,d); IR(KBr,cm⁻¹): 3112,1597,1574,1487,1458,1406,1346,1294,1061,1022,970,912 ,860,742; MS(FAB,MH⁺): 477; Melting point: 139-140°C (decomposed)

### Example 81

¹H-NMR(DMSO-d₆)
4.70(1H,d),4.73(1H,d),5.00(2H,t),6.19(2H,s),7.04(1H,d),7.13(1H,dd),7.19(1H,d),7.48(1H,d),8.46(1H,d); IR(KBr,cm⁻¹): 3120,1593,1569,1483,1427,1352,1299,1060,1027,982,902,873,744; MS(FAB,MH⁺): 377; Melting point: 80-83°C (decomposed)

### Test 1 (Proton Pump Inhibitory Effect)

### (1) Preparation of H⁺/K⁺-ATPase enzyme sample

A swine stomach which had been stored frozen was separated into a muscularis and a mucosal layer. After the mucosal layer was disrupted with a mixer in 5 volumes of 20 mmol/L tris-HCl buffer containing 0.25 mol/L of sucrose and 1 mmol/L of EGTA (pH 7.4, hereafter abbreviated as "tris-HCl buffer"), centrifugation was conducted at 9,000 × g for 30 minutes. The supernatant was gently overlaid on 20 mmol/L tris-HCl buffer containing 30% of sucrose and 1 mmol/L of EGTA (pH 7.4, 8 mL), followed by ultracentrifugation at 100,000 × g for 60 minutes. An interface fraction obtained by the centrifugation was collected and subjected to centrifugation twice under 113,000 × g for 60 minutes. The sediment was suspended with tris-HCl buffer, and the suspension was homogenized at low rotational speed to provide an enzyme sample. The enzyme sample was stored frozen at -80°C. Using the method reported by Smith et al. (Anal. Biochem., 150, 76-85 (1985)), the thus-obtained sample was quantitatively analyzed for its protein content with a BCA protein assay reagent. The above procedure was all conducted under water cooling.

### (2) Discussion about H⁺/K⁺-ATPase enzyme inhibiting activity at pH 1

Following a method making use of a method for the quantitation of inorganic phosphorus as a degradation product (Biochem. Biophys. Res. Commun., 40, 880-886 (1970)), an assay of H⁺/K⁺-ATPase enzyme inhibiting activity was performed by using ATP as a substrate. As a hydrochloric acid solution for the pretreatment of each test compound, a 1×10⁻¹ mol/L (pH 1) solution of hydrochloric acid with 50% DMSO contained therein was used. Solutions of each test compound at various concentrations were added to aliquots of the hydrochloric acid solution such that the solutions were diluted 1, 000-fold, and the thus-diluted solutions were left over at room temperature for 15 minutes. Subsequent to the above-described pretreatment, 10-µL aliquots of the solutions were added to 840-µL aliquots of 40 mmol/L tris-acetate buffer (pH 7.5; contained 2 mmol/L of MgCl₂; hereinafter abbreviated as the "TE buffer") which contained or did not contain 20 mmol/L of KCl. 840-µL Aliquots of an enzyme solution (5 µg proteins) diluted with the TE buffer were then added. After incubated at 37°C for 30 minutes, 50-µL aliquots of 40 mmol/L ATP solution (dissolved in the TE buffer free of KCl) were added to initiate an enzymatic reaction (total volume: 1 mL, final ATP concentration: 2 mmol/L). Subsequent to incubation at 37°C for 30 minutes, 2-mL aliquots of ice-cold 12% trichloroacetic acid were added to terminate the enzymatic reaction. A molybdenum reagent (1 mL; 3.75% ammonium molybdate/1.5 mol/L sulfuric acid) and butyl acetate (5 mL) were added. After vigorous shaking and mixing for 5 minutes, the absorbance of a butyl acetate layer was measured at 310 nm. From absorbances obtained by dissolving KH₂PO₄ solutions of various concentrations in aliquots of an 8% TCA solution andperforming a similar procedure, a standard curve was prepared to perform a conversion of each quantity of inorganic phosphorus. The assay was conducted in duplicate. From a difference between an average quantity of inorganic phosphorus in the case of the KCl-containing TE buffer and an average quantity of inorganic phosphorus in the case of the KCl-free TE buffer, a residual activity was determined. Supposing that the activity of the control value (DMSO) was 100%, the percent inhibition of the compound was calculated. As the inhibition potency of each test compound, an IC₅₀ value was calculated and presented. The test compounds were al used by dissolving them in dimethyl sulfoxide immediately before their use.

The results so obtained are presented in Table 10.

**Table 10**

| Test compound | Proton pump inhibitory effect IC₅₀ (µmol/L) |
|---|---|
| Compound of Example 47 | 4.85 |
| Compound of Example 49 | 1.03 |
| Compound of Example 50 | 0.87 |
| Compound of Example 51 | 3.32 |
| Compound of Example 54 | 3.88 |
| Compound of Example 64 | 2.28 |
| Compound of Example 68 | 3.2 |
| Omeprazole | 1.01 |
| Esomeprazole | 2.06 |
| Lansoprazole | 1.61 |
| Pantoprazole | 5.46 |
| Rabeprazole | 1.61 |

### Test 2 (Gastric acid secretion inhibitory effect)

(1) As laboratory animals, seven-week old male Sprague-Dawley (SD) rats were purchased from CHARLES RIVER JAPAN, INC. Subsequent to preliminary rearing for 5 days or longer, they were used in an experiment.
   Each test medicine was suspended or dissolved in a 0.5% carboxymethylcellulose sodium solution to prepare a volume of 2.5 mL/kg.
(2) Measurement of gastric acid secretion
   Using rats fasted for 18 hours, histamine-stimulated gastric acid secretion in an acute fistula method following the method reported by Hiramatsu et al. (Dig. Dis. Sci., 39, 689-697 (1994)) was measured. Described specifically, each rat was lightly anesthetized with diethyl ether. After that, a winged tube needle with 3.8% citric acid solution filled therein was caused to dwell in the caudal vein. Celiotomy was performed, and the pyloric part of the stomach was ligated. A small hole was then formed in the duodenum, and a feeding tube with physiological saline filled therein was caused to dwell there. After the interior of the stomach was washed with physiological saline, a polyethylene-made fistula tube (inner diameter: 4 mm) was fitted to the proventriculus part and an incised part of the proventriculus was ligated to hold the fistula tube in place. The stomach and duodenum were returned into the abdominal cavity. Celiotomy was performed with the fistula tube and feeding tube being left extending outside, and the rat was placed in a Ballman Cage Type II. Histamine was continuously injected through the winged needle allowed to dwell in the caudal vein (8 mg/kg/h, 1.38 mL/h). Fifteenminutes later, the gastric juice was sampled, and after that, the gastric juice was sampled at intervals of 1 hour. After the amount of each gastric juice sample was recorded, it was titrated with 0.1 mol/L NaOH to a final point of pH 7.0. From the volume required for the titration, the acidity was calculated. The quantity of the secreted gastric acid was determined from the product of the amount of the gastric juice and the acidity. Incidentally, the rats were divided into groups such that the total amount of the first gastric juice samples became equal in each group. Each test medicine was administered in a volume of 2.5 mL/kg into the duodenum through the feeding tube fitted beforehand in the duodenum. To a control group, 0.5% Na-CMC solution was administered.
(3) Analysis of the results

From the results so obtained, data were presented in terms of average ± S.D. The sum of quantities of gastric acid secreted on the 3^{rd}, 4^{th} and 5^{th} hours after the first sampling was recorded as a total quantity of secreted gastric acid. The percent inhibition in each group was calculated from the total quantity of secreted gastric acid in the control group and the total quantity of secreted gastric acid in the group. From the percent inhibition in each group, the 50% effective dose (ED₅₀) was next calculated by the Probit method. The results are presented in Table 11.

**Table 11**

| Test compound | Gastric acid secretion inhibitory effect ED₅₀ (mg/kg) |
|---|---|
| Compound of Ex. 25 | 1.1 |
| Compound of Ex. 26 | 2.6 |
| (+) Isomer of Ex. 36 | 1.1 |
| Compound of Ex. 51 | 2.4 |
| Compound of Ex. 55 | 3.6 |
| Compound of Ex. 71 | 2.5 |
| Compound of Ex. 72 | 1.6 |
| Compound of Ex. 73 | 1.8 |
| Omeprazole | 3.6 |
| Esomeprazole | 2.9 |
| Lansoprazole | 1.1 |
| Pantoprazole | 0.34 |
| Rabeprazole | 2.5 |

### Test 3 (Human CPY2C19 Activity Inhibition Test)

CYP2C19 specifically induces the metabolic reaction of from S-(+)-mephenytoin into 4'-hydroxymephenytoin. Using the CYP2C19 expression system, the inhibitory effect of each test substance was investigated based on the amount (concentration) of formed 4'-hydroxymephenytion as an index of CYP2C19 activity.

### a) Metabolic activity test

As a reaction mixture, a 125 µmol/L solution of S-(+)-mephenytoin in methanol (50 µL) and a 25 µmol/L solution of each test substance in methanol (0, 10, 100 or 200 µL) were added to 10-mL test tube made of glass, and the solvent was distilled off under a nitrogen gas stream (40°C). A 20:10 mixture (75 µL) of 0.5 M phosphate buffer (pH 7.4) and 0.5 mM EDTA, purified water (140 µL) and microsomes (10 µL) from a human CYP2C19 enzyme expression system (purchased from Sumitomo Chemical Co., Ltd.) were added, followed by the addition of an NADPH-generating system (25 µL, a mixture of 60 mM MgCl₂, 6.7 mg/mL β-NADP⁺, 27.2 mg/mL G-6-P and 10 µL G-6-Pdh) to initiate a reaction.

After incubated at 37°C for 10 minutes, a 12% aqueous solution of perchloric acid (50 µL) was added to terminate the reaction.

### b) Quantitation method

Subsequent to the termination of the reaction, 10 µg/mL methyl p-hydroxybenzoate (50µL) as an internal standard (I.S.) and diethyl ether (2 mL) were added. The resulting mixture was shaken for 10 minutes, and then centrifuged (3,000 rpm, 10 minutes). The organic layer was separately collected, and under a nitrogen gas stream (40°C), the solvent was distilled off. The residue was dissolved in a mobile phase (200 µL), and an analysis was conducted by high performance liquid chromatography (HPLC).

Using a solution of 4'-hydroxymephenytoin in lieu of the S-(+)-mephenytoin solution, a calibration line was prepared following the above-described reaction method. Quantitation was conducted based on a peak area ratio of 4'-hydroxymephenytoin to the I.S. Comparing the formed amount of 4'-hydroxymephenytoin as a control with that of 4'-hydroxymephenytoin at the time of the existence of the test compound, the comparison result was used as an index of activity. The results upon addition of the test compounds at concentration of 10 µM are presented in Table 12.

**Table 12**

| Test compound | CYP2C19 activity inhibition (%) |
|---|---|
| Compound of Example 21 | 78.6 |
| Compound of Example 23 | 75.6 |
| Compound of Example 24 | 81.9 |
| Compound of Example 26 | 73.2 |
| Compound of Example 33 | 62.1 |
| Racemic mixture of Example 36 | 61.7 |
| (+) Isomer of Example 36 | 70.6 |
| Compound of Example 48 | 72.3 |
| Compound of Example 55 | 63.8 |
| Racemic mixture of Example 57 | 73 |
| (+) Isomer of Example 57 | 67.4 |
| (-) Isomer of Example 57 | 77.6 |
| Compound of Example 58 | 67.3 |
| Compound of Example 59 | 61.7 |
| Compound of Example 60 | 71.4 |
| Compound of Example 61 | 64.8 |
| Compound of Example 62 | 95.3 |
| Compound of Example 63 | 72.1 |
| Compound of Example 65 | 76.7 |
| Compound of Example 72 | 65 |
| Compound of Example 73 | 75.7 |
| Compound of Example 78 | 62 |
| Omeprazole | 18.4 |
| Esomeprazole | 35.4 |
| Lansoprazole | 8.7 |
| Pantoprazole | 33.1 |
| Rabeprazole | 39.8 |

HPLC was conducted under the following HPLC conditions A or HPLC conditions B.

### <Measurement conditions>

### HPLC conditions A:

Column: "CAPCELL PAK C18 UG120", 5 µm, 4.6 mm in diameter × 250 mm
Precolumn: "CAPCELL PAK C18 UG120", 4.0 mm in diameter × 10 mm
Detection wavelength: UV 204 nm
Detector sensitivity: 0.01 AUSF
Mobile phase: CH₃CN: 50 mM phosphate buffer (pH 8) = 20 : 80
Flow rate of the mobile phase: 0.8 mL/min
I.S.: methyl p-hydroxybenzoate
Column temperature: 40°C
Injection volume: 40 µL

### HPLC conditions B:

Column: "CAPCELL PAK C18 UG120", 5 µm, 4.6 mm in diameter × 250 mm
Precolumn: "CAPCELL PAK C18 UG120", 4.0 mm in diameter × 10 mm
Detection wavelength: UV 204 nm
Detector sensitivity: 0.01 AUSF
Mobile phase: CH₃CN: 50 mM phosphate buffer (pH 4) =20:80
Flow rate of the mobile phase: 0.8 mL/min
I.S.: methyl p-hydroxybenzoate
Column temperature: 40°C
Injection volume: 40 µL

### Test 4 (Human CYP1A Induction Test)

CYP1A specifically induces the metabolic reaction of from 7-ethoxyresorfin into resorfin. Using HepG2 cells, an investigation was hence made about CYPIA induction effect based on the formation of resorfin upon exposure to a test substance as an index of CYP1A activity.

### (a) Exposure of HepG2 Cells and Preparation of Samples

After HepG2 cells (purchased from DAINIPPON PHARMACEUTICAL CO., LTD.) were incubated for about 7 days in a medium containing inactivated calf serum (minimum essential medium (450 mL) containing 100 mM sodium pyruvate (5 mL), nonessential amino acids (× 100) (5 mL) , an antibacterial and antifungal solution (10,000 units of penicillin, 10 mg of streptomycin and 25 µg of amphotericin B per mL), a 200 mL L-glutamine solution (5 mL), and inactivated calf serum (50 mL)) (70 to 80% confluence), the medium was removed under suction. Subsequent to the addition of a fresh supply of the medium (10 mL) and a solution (5 µL) of a test substance in dimethylsulfoxide (DMSO), the cells were incubated at 37°C for 24 hours. The incubation was conducted by setting the concentration of DMSO at 0.05%, the concentration of the test substance at 20 µM, the concentrations of β-naphtoflavone and 3-methylcholanthrene as positive control substances at 20 µM and 0.1 µM, respectively, in the culture medium. After 24 hours of the incubation, the medium was removed under suction, and the cells were washed twice with phosphate buffer (5 mL) warmed to 37°C. Subsequently, awater-cooled, 3-folddilution (4 to 5 mL) of a 20:10 mixture of 0.5 M phosphate buffer (pH 7.4) and 0.5 mM EDTA was added. The cells were separated with a cell scraper, and were then homogenized with a glass homogenizer under ice cooling to provide as samples for the following tests.

### (b) Metabolic activity test

A 78 µM solution (10 µL) of ethoxyresorfin in DMSO was added to the cell homogenate (890 µL), followed by the addition of an NADPH-generating system (a mixture of 60 mM MgCl₂, 6.7 mg/mL β-NADP⁺, 27.2 mg/mL G-6-P, 10 µL G-6-Pdh) (100 µL). Immediately, a reaction was initiated at 37°C.

Subsequent to incubation at 37°C for 10 or 20 minutes, a 17% aqueous solution of potassium carbonate (100 µL) was added to terminate the reaction.

### (c) Quantitation method

After the termination of the reaction, centrifugation was conducted (3,000 rpm, 20 minutes) and the resorfin in the supernatant was measured by a fluorophotometer (excitation: 550 nm, emission: 586 nm). Following the above-described reaction method, a calibration line was prepared using a resorfin solution.

The concentration of proteins in the sample was measured by a spectrophotometer (wavelength: 595 nm) after adding a 5-fold dilution (2.5 mL) of a protein measurement solution (Bio-Rad) to the cell homogenate (100 µL) and allowing the resultant mixture at room temperature for 30 minutes. A calibration line for protein concentrations was prepared using human albumin (product of Sigma Chemical Company).

By dividing the concentration of the produced resorfin with the metabolic reaction time and the concentration of proteins in the sample, the metabolism rate was calculated. Supposing that the rate of formation of resorfin under exposure to omeprazole as a positive control substance was 100%, the rate of formation under exposure to the test substance was expressed as an index of CYP1A induction activity. The results are presented in Table 13.

**Table 13**

| Test compound | CYP1A2 induction ability (%) |
|---|---|
| Compound of Example 33 | 9.1 |
| Compound of Example 47 | 9.7 |
| Compound of Example 51 | 3.7 |
| Compound of Example 53 | 5.7 |
| Compound of Example 54 | 8.9 |
| Compound of Example 55 | 6.6 |
| Compound of Example 60 | 4.45 |
| Compound of Example 61 | 5.2 |
| Compound of Example 65 | 8.7 |
| Compound of Example 70 | 9 |
| Compound of Example 71 | 7.9 |
| Compound of Example 73 | 6.24 |
| Compound of Example 78 | 8.3 |
| Omeprazole | 100 |
| Esomeprazole | 91.3 |
| Lansoprazole | 47.1 |
| Pantoprazole | 14.5 |
| Rabeprazole | 344.2 |

As evident from Tables 10 to 13, the invention compounds have excellent proton pump inhibitory effect and gastric acid secretion inhibiting effect. As the index of CYP2C19 activity is as high as 60% or greater, CYP2C19 does not contribute much to the metabolism of the invention compounds so that individual differences in therapeutic effects are small. It is also appreciated that the invention compounds are high in safety, because their CYP1A2 induction activities are about 10% or lower and are extremely low. In contrast, the conventional proton pump inhibitors have extremely low indexes of CYP2C19 activity of less than 40%. It is, therefore, appreciated that CYP2C19 significantly contributes to their metabolism and individual differences arise in their therapeutic effects. It has also been found that most of the conventional proton pump inhibitors have extremely high CYP1A2 induction activity.

### Industrial Applicability

The compounds according to the present invention do not bring about much individual differences in therapeutic effects despite the existence of individual differences in the CYP2C19 activity. At the same dose, they can hence bring about appropriate therapeutic effects for all patients. In addition, they are low in the risk of induction of an interaction or a cancer caused by induction of the CYP1A family. Accordingly, they are useful as pepticulcer therapeutic agents which are safe and surely bring about therapeutic effects, and also as gastric acid secretion inhibitors in *Helicobacter* *pylori* eradication therapy.

## Claims

1. A 1-N-aminobenzoimidazole derivative represented by the following formula (I): 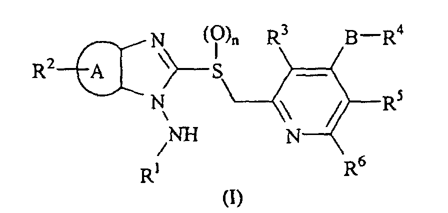 wherein:
R¹ represents an alkyl group optionally substituted by one or more substituents which may be the same or different and which are selected from halogen atoms, hydroxy groups, phenyl groups, hydroxyphenyl groups, amino groups, alkoxy groups, alkoxycarbonyl groups or alkylamino groups, analkenyl group, an acyl group, an alkoxycarbonyl group, a benzyloxycarbonyl group, a formyl group, a phenyl group, or a hydrogen atom;
R² represents an alkyl group which may be substituted by a hydroxy or alkoxycarbonyl group, an acyl group which may be substituted by a halogen atom, a cyano group, a carboxyl group, an alkoxycarbonyl group, or a hydrogen atom;
R³, R⁵ and R⁶ may be the same or different and each represents an alkyl group, an alkoxy group or a hydrogen atom;
R⁴ represents an alkyl group optionally substituted by one or more substituents which may be the same or different and which are selected from halogen atoms, hydroxy groups, alkyl groups which may be substituted by 1 to 8 halogen atoms, alkoxy groups which may be substituted by 1 to 8 halogen atoms, furyl groups or morpholino groups, or a geranyl group;
A represents a benzene ring, a pyridine ring, or a thiophene ring;
B represents an oxygen atom or a sulfur atom; and
n stands for an integer of from 0 to 2; or a salt thereof.

2. The 1-N-aminobenzimidazole derivative or a salt thereof according to claim 1, wherein in said formula (I),
R¹ is a C₁₋₆ alkyl group optionally substituted by 1 or more substituents which may be the same or different and which are selected from halogen atoms, hydroxy groups, phenyl groups, hydroxyphenyl groups, amino groups, C₁₋₆ alkoxy groups, C₁₋₆ alkoxycarbonyl groups or C₁₋₆ alkoxyamino groups, a C₂₋₆ alkenyl group, a C₂₋₆ acyl group, a C₁₋₆ alkoxycarbonyl group, a benzyloxycarbonyl group, a formyl group, a phenyl group, or a hydrogen atom;
R² is a C₁₋₆ alkyl group which may be substituted by a hydroxyl group or a C₁₋₆ alkoxycarbonyl group, a C₂₋₆ acyl group which may be substituted by a halogen atom, a cyano group, a carboxyl group, a C₁₋₆ alkoxycarbonyl group, or a hydrogen atom;
R³, R⁵ and R⁶ may be the same or different and are each a C₁₋₆ alkyl group, a C₁₋₆ alkoxy group or a hydrogen atom;
R⁴ is a C₁₋₆ alkyl group optionally substituted by one or more substituents which may be the same or different and which are selected from halogen atoms, hydroxy groups, C₁₋₆ alkyl groups which may be substituted by 1 to 8 halogen atoms, C₁₋₆ alkoxy groups which may be substituted by 1 to 8 halogen atoms, furyl groups or morpholino groups, or a geranyl group;
A represents a benzene ring, a pyridine ring, or a thiophene ring;
B represents an oxygen atom or a sulfur atom; and
n stands for an integer of from 0 to 2.

3. The 1-N-aminobenzimidazole derivative or a salt thereof according to claim 1, wherein in said formula (I),
R¹ is an unsubstituted C₁₋₆ alkyl group, a C₁₋₆ alkyl group substituted by three halogen atoms, a C₁₋₆ alkyl group substituted by one hydroxy, phenyl or hydroxyphenyl group, a C₁₋₆ alkyl group substituted by one C₁₋₆ alkoxycarbonyl group, a C₁₋₆ alkyl group substitutedby one di-(C₁₋₆ alkyl)amino group, a C₂₋₆ alkenyl group, an allyl group, or a phenyl group;
R² is a C₁₋₆ alkyl group substituted by one hydroxy or C₁₋₆ alkoxycarbonyl group, a C₂₋₆ acyl group substituted by two halogen atoms, a cyano group, a carboxyl group, a C₁₋₆ alkoxycarbonyl group, or a hydrogen atom;
R³, R⁵ and R⁶ may be the same or different and are each a C₁₋₆ alkyl group, a C₁₋₆ alkoxy group or a hydrogen atom;
R⁴ is a C₁₋₆ alkyl group optionally substituted by one or more substituents which may be the same or different and which are selected from halogen atoms, hydroxy groups, C₁₋₆ alkyl groups which may be substituted by 1 to 8 halogen atoms, C₁₋₆ alkoxy groups which may be substituted by 1 to 8 halogen atoms, furyl groups or morpholino groups, or a geranyl group;
A represents a benzene ring;
B represents an oxygen atom; and
n is 1.

4. The 1-N-aminobenzimidazole derivative or a salt thereof according to claim 1, wherein in said formula (I),
R¹ is a methyl, ethyl, propyl, isopropyl, isobutyl, hexyl, 2-hydroxyethyl, 2-hydroxypropyl, 3-hydroxypropyl, 4-hydroxybutyl, 5-hydroxypentyl, 2,2,2,-trifluoroethyl, 2-phenethyl, benzyl, allyl, p-hydroxybenzyl, 2-hydroxy-2-phenethyl, 2-dimethylaminoethyl, methoxycarbonylmethyl or phenyl group;
R² is a methoxy, difluoromethoxy, cyano, methoxycarbonyl, methoxycarbonylmethyl, carboxyl or hydroxymethyl group, or a hydrogen atom;
R³ is a methyl or methoxy group, or a hydrogen atom;
R⁴ is a methyl, ethyl, propyl, isopropyl, butyl, isobutyl, hexyl, octyl, 2-methoxyethyl, 3-methoxypropyl, 2,2,2-trifluoroethyl, 4,4,4-trifluorobutyl, 2,2,3,3,4,4,4-heptafluorobutyl, 2-(2,2,2-trifluoroethoxy)ethyl, 3-(2,2,2-trifluoroethoxy)propyl, 2-hydroxyethyl or geranyl group;
R⁵ is a methyl group or a hydrogen atom;
R⁶ is a hydrogen atom;
A is a benzene ring;
B is an oxygen atom; and
n is 1.

5. The medicine comprising a compound according to any one of claims 1-4 or a salt thereof.

6. The medicine according to claim 5, which is a peptic ulcer therapeutic agent.

7. A medicinal composition comprising a compound according to any one of claims 1-4 and a pharmacologically acceptable carrier.

8. Use of a compound according to any one of claims 1-4 for the production of a medicine.

9. Use according to claim 8, wherein said medicine is a peptic ulcer therapeutic agent.

10. A method for the treatment of a peptic ulcer, which comprises administering an effective amount of a compound according to any one of claims 1-4.
